# FASCICULE DE BREVET EUROPEEN

(11) **EP 1 558 637 B1**
(45) Date de publication et mention de la délivrance du brevet: **04.11.2009**
(21) Numéro de dépôt: 03767905.7
(22) Date de dépôt: 04.11.2003
(51) Int. Cl.: C07K 14/315, C12N 15/31, C12Q 1/68, C12Q 1/04

(54) **IDENTIFICATION MOLECULAIRE DES BACTERIES DU GENRE STREPTOCOCCUS ET GENRES APPARENTES**
MOLEKULARE IDENTIFIZIERUNG VON BAKTERIEN DES GENUS STREPTOCOCCUS UND VERWANDTE GENUS
MOLECULAR IDENTIFICATION OF BACTERIA OF GENUS STREPTOCOCCUS AND RELATED GENUSES

(30) Priorité: 05.11.2002 FR 0213792
(43) Date de publication de la demande: 03.08.2005
(73) Titulaire: Université de la Méditerranée, Aix-Marseille II, 13284 Marseille Cedex 07 (FR); CENTRE NATIONAL DE LA RECHERCHE SCIENTIFIQUE (CNRS), 75794 Paris Cedex 16 (FR)
(72) Inventeur: RAOULT, Didier, F-13008 Marseille (FR); DRANCOURT, Michel, F-13012 Marseille (FR)
(74) Mandataire: Domange, Maxime
(86) Numéro de dépôt international: PCT/FR2003/003293
(87) Numéro de publication internationale: WO 2004/041841

(56) Documents cités:
- WO-A-02/077021
- US-B1- 6 420 135
- ENRIGHT M ET AL: "Molecular Evolution of Rifampicin Resistance in Streptococcus pneumoniae" MICRIBIAL DRUG RESISTANCE, vol. 4, no. 1, 1998, pages 65-70, XP009017720
- DATABASE EMBL [Online] 16 avril 2001 (2001-04-16), XP002255355 Database accession no. AE006480; AE004092
- DATABASE EMBL [Online] 28 mars 2002 (2002-03-28), XP002255356 Database accession no. AE009961; AE009949
- DATABASE EMBL [Online] 7 septembre 2001 (2001-09-07), XP002255357 Database accession no. AE008542; AE007317
- DATABASE EMBL [Online] 2 septembre 2002 (2002-09-02), XP002255358 Database accession no. AE014199; AE009948
- DATABASE EMBL [Online] 27 octobre 2002 (2002-10-27), XP002255359 Database accession no. AE015022; AE014133
- DATABASE EMBL [Online] 28 septembre 1999 (1999-09-28), XP002255360 Database accession no. Y16468
- DATABASE EMBL [Online] 3 octobre 2000 (2000-10-03), XP002255361 Database accession no. Y16469

## Description

La présente invention concerne le domaine du diagnostic. Plus précisément, l'invention concerne une méthode pour l'identification moléculaire des bactéries du genre *Streptococcus* et genres apparentés *Enterococcus, Gemella, Abiotrophia* et *Granulicatella* par les techniques de détection et/ou d'amplification et séquençage à l'aide de sondes ou d'amorces oligonucléotidiques appliquées à des souches de ces genres bactériens.

Les bactéries du genre *Streptococcus* et de quatre genres apparentés : *Enterococcus, Gemella, Abiotrophia* et *Granulicatella,* sont des bactéries cocciformes, gram positif et catalase négative dont on reconnaît actuellement plus d'une quarantaine d'espèces. Les bactéries du genre *Lactococcus*, précédemment classées parmi les streptocoques comme *Streptococcus* groupe N, n'entrent pas dans le champ de ce brevet du fait de leur rareté en pathologie humaine, et du fait qu'elles sont facilement discriminés des streptocoques par leur croissance à + 10°C. Le genre *Streptococcus* comporte officiellement 55 espèces. Le genre *Gemella* comporte 6 espèces, le genre *Abiotrophia* comporte 1 espèce, le genre *Granulicatella* comporte 3 espèces, le genre *Enterococcus* comporte 24 espèces [www.springer-ny.com/bergeysoutline/main.htm]. Ces espèces sont facilement et fréquemment cultivées à partir de prélèvements environnementaux, de prélèvements cliniques vétérinaires et de prélèvements cliniques humains [Ruoff KI. (1999) in Manuel of Clinical Microbiology, pp. 283-296, ASM press]. Chez l'homme, différentes espèces du genre *Streptococcus* sont responsables d'infections communautaires éventuellement sévères du fait du caractère invasif des streptocoques considérés ou du fait de la production de toxines et de manifestations cliniques éventuellement graves à distance du foyer infectieux. Par exemple, *Streptococcus pyogenes* (Streptocoque groupe A) est responsable d'angines et de syndromes post-streptococciques incluant le rhumatisme articulaire aigu au cours duquel la destruction des valves cardiaques par un processus inflammatoire est responsable d'une valvulopathie éventuellement mortelle. Egalement, plusieurs espèces du genre *Streptococcus* en particulier les Streptocoques du groupe A, du groupe C, et du groupe G sont responsables d'infections invasives mortelles en particulier de myosite c'est-à-dire de destruction des tissus cutanés et sous cutanés et du tissu musculaire comme cela a été décrit depuis quelques années. Egalement par exemple *Streptococcus pneumoniae* (pneumocoque) est responsable de pneumonie, de méningite et de septicémie. Par ailleurs, les bactéries des genres *Streptococcus, Enterococcus, Gemella, Abiotrophia,* et *Granulicatella* sont responsables d'endocardites c'est-à-dire d'infection des valves cardiaques chez l'homme, lesquelles constituent des maladies infectieuses mortelles [Casalta JP et al. Journal Clinical Microbiology, 2002, 40: 1845-1847]. Egalement, certaines espèces des genres considérés sont responsables d'infections nosocomiales, par exemple, les bactéries du genre *Streptococcus* du groupe A sont responsables de bactériémies qui succèdent à des explorations par endoscopie digestive. Egalement, les bactéries du genre *Enterococcus* sont responsables d'infections urinaires nosocomiales après utilisation d'antibio-prophylaxie par des antibiotiques de la famille des céphalosporines auxquelles elles sont naturellement résistantes. Ces espèces bactériennes posent par ailleurs le problème de leur résistance croissante aux antibiotiques, résistance à la pénicilline G de *Streptococcus pneumoniae* [Garav J. Lancet Infect. Dis. 2002, 2 : 404-415] et résistance à la vancomycine d'*Enterococcus* spp. [Gold H.S. Clin. Infect. Dis. 2001, 33 : 210-219 ; Bonten M.J. et al. Lancet Infect. Dis. 2001, 1 : 314-325].

Ces différentes espèces bactériennes posent le problème de leur détection dans les prélèvements pathologiques chez l'homme et de leur identification lorsqu'elles ont été isolées à partir desdits prélèvements. Les méthodes conventionnelles de détection reposent en effet sur la mise en évidence de bactéries cocciformes gram positif, à l'examen direct du produit pathologique. II est cependant connu que cette détection microscopique des bactéries du genre *Streptococcus* et de genres apparentés dans les prélèvements cliniques a un seuil de sensibilité de 10⁴ CFU/ml. Il est donc tout à fait possible qu'un prélèvement pathologique chez l'homme ou chez l'animal contienne une des espèces considérées qui ne soit pas détectée à l'examen microscopique direct de ce prélèvement pathologique. Par ailleurs, bien que leur structure soit celle de bactéries Gram - positif, elles peuvent apparaître faussement Gram-négatif après coloration de Gram du prélèvement pathologique et donner lieu à une identification erronée ou à une impasse d'identification. Ceci est particulièrement fréquent pour les bactéries du genre *Gemella*. Chez l'homme, c'est en particulier le cas lors de l'examen anatomopathologique et bactériologique des valves cardiaques dans le cas d'une endocardite.

Lorsque qu'une bactérie d'une espèce des genres considérés est isolée au laboratoire, les méthodes conventionnelles d'identification phénotypique sont les plus couramment utilisées pour l'identification des bactéries des espèces du genre *Streptococcus* et des genres apparentés et plusieurs trousses d'identification ainsi que des automates ont été développés pour aider à l'identification phénotypique des bactéries du genre *Streptococcus* et des genres apparentés. Sur ce plan, le degré d'identification en pratique courante est variable. En particulier, un des tests utilisés pour l'identification des Streptocoques et des bactéries des genres apparentés est l'observation d'une réaction hémolytique, c'est-à-dire la destruction par la bactérie des hématies contenues dans une-gélose au sang. Cependant cette réaction d'hémolyse peut être inhibée par la présence d'oxygène ou par la présence de péroxyde lorsque les bactéries Streptocoques sont cultivées en présence de concentration importante de dioxyde de carbone. Il est par ailleurs reconnu qu'il existe un certain degré de subjectivité dans l'appréciation de l'hémolyse par les colonies de Streptocoques et donc une variabilité d'inter opérateur qui nuit ensuite à la qualité de l'identification de ces bactéries. Pour les streptocoques alpha-hémolytiques, un deuxième test est celui de la sensibilité à l'optochine qui permet de reconnaître *Streptococcus pneumoniae* qui est sensible à ce composé. Cependant, des souches de *Streptococcus pneumoniae* résistant à l'optochine ont été rapportées [Lund E. Acta Patho. Microbiol. Immunol. Scand. 1959, 47, 308-315]. Un dernier test phénotypique est le sérotypage, ce test peut être faussement positif en particulier pour les streptocoques de sérogroupe D du fait d'antigénicité croisée entre les streptocoques du groupe D, *Enterococcus* et *Pediococcus.*

Plusieurs systèmes moléculaires ont été développés pour l'identification de certains sérogroupes ou de certaines espèces du genre *Streptococcus,* en particulier les streptocoques du groupes A (Streptococcus pyogenes, Streptococcus anginosus, Streptococcus constellatus, Streptococcus intermedius) et du groupe B (Streptococcus agalactiae) [Daly J.A. et al. J Clin Microbiol. 1991, 29 : 80-82 ; Heelan J.S. et al. Diagn. Microbiol. Infect. Dis. 1996, 24 : 65-69] de même que pour *Streptococcus pneumoniae* [Denys G.A. et Carrey R.B. J. Clin. Microbiol. 1992,30 : 2725-2727] par hybridation de sondes spécifiques ciblant le gène codant l'ARN ribosomal 16S. Egalement, différents systèmes basés sur l'amplification par PCR de gènes codant pour des toxines ou des facteurs de virulence ont été développés pour la discrimination de *Streptococcus pneumoniae* parmi les Streptocoques α-hémolytiques [Salo P. et al. J. Infect. Dis. 1995, 171 : 479-482 ; Morrisson K. et al. J. Clin. Microbiol. 2000, 38, 434-437 ; Kaijalainen T. et al. J. Microbiol. Meth. 2002, 51 : 111-118], ainsi que pour la détection de *Streptococcus agalactiae* [Mawn J.A. et al. J. Clin. Pathol. 1993, 46 : 633-636]. Ces différents systèmes cependant ne permettent l'identification que d'une ou quelques espèces du genre *Streptococcus.*

Un système d'identification de trois espèces de streptocoque a été développé, basé sur l'amplification de l'entretoise 16S-23S [Forsman P. et al. Microbiology, 1997, 143, 3491-3500], mais l'identification n'a-été limitée dans ce travail qu'à certaines espèces d'intérêt animal: *Streptococcus agalactiae, Streptococcus dysgalactiae* et *Streptococcus uberis.* Par ailleurs, il est actuellement indispensable de disposer dans les laboratoires de 2 cibles moléculaires distinctes pour la détection et l'identification des streptocoques, ceci afin de pallier les risques de contamination moléculaire inhérents à l'utilisation d'une seule cible.

Enfin, aucun système de détection et d'identification des genres apparentés à *Streptococcus* n'a été développé et plus particulierement pour les bacteries du genre *Enterococcus, Gemella, Abiotrophia* et *Granulicatella.*

Les inventeurs ont démontré selon la présente invention, que le gène *rpoB* constitue un marqueur génétique permettant la détection et l'identification spécifique de la bactérie de chaque espèce du genre *Streptococcus* et de 4 genres apparentés : *Enteroccocus, Gemella, Abiotrophia* et *Granulicatella.*

Bien que ce gène ait été précédemment montré comme un outil d'identification bactérienne dans différents genres bactériens, aucune publication ne fait mention de son utilisation pour l'identification des bactéries des genres *Streptococcus* et des quatre genres apparentés et il n'y avait donc aucune suggestion quant à l'intérêt de la séquence de ce gène pour l'identification des dites bactéries. Au contraire, quelques séquences partielles du gène *rpob* chez quelques espèces, disponibles dans GenBank montrait une faible hétérogénéité, faisant douter de l'intérêt de ce gène comme outil d'identification pour ces bactéries. Enfin, les inventeurs ont développé un outil d'identification de quatre genres bactériens simultanément, obligeant la mise au point d'amorces dégénérées qui ne pouvaient être déduites d'aucune des séquences *rpo*B déterminées pour chaque espèce.

Plus particulièrement, la présente invention concerne des séquences d'acides nucléiques spécifiques du genre ou de chaque espèce du genre *Streptococcus* et des genres apparentés dont la séquence nucléotidique est tirée du gène *rpoB* des dites bactéries.

Selon Lazcano et al. [J. Mol. Evol. (1988) 27:365-376], les ARN polymérases sont divisées en deux groupes selon leur origine, l'un constitué par les ARN polymérases virales ARN- ou ADN-dépendantes, et l'autre constitué par les ARN polymérases ADN-dépendantes d'origine eucaryote ou procaryotes (archaébactéries et eubactéries). Les ARN polymérases ADN-dépendantes eubactériennes sont caractérisées par une constitution multimérique simple et conservée notée « core enzyme », représentée par αββ', ou « holoenzyme » représentée par αββ'σ [Yura and Ishihama, Ann. Rev. Genet. (1979) 13 :59-97].

De nombreux travaux ont mis en évidence le rôle fonctionnel, au sein du complexe enzymatique multimérique, de la sous-unité β de l'ARN polymérase eubactérienne. Les ARN polymérases archaébactérienne et eucaryote présentent, pour leur part, une structure plus complexe pouvant atteindre une dizaine, voire une trentaine de sous-unités [Pühlet et al. Proc. Natl. Acad. Sci. USA (1989) 86 :4569-4573].

Les gènes qui codent les différentes sous-unités αββ'σ de l'ARN polymérase ADN-dépendante chez les eubactéries, respectivement les gènes *rpoA, rpoB, rpoC* et *rpoD,* sont classés en différents groupes comprenant les gènes codant pour des protéïnes constitutives des sous-unités ribosomiques ou pour des enzymes impliqués dans la réplication et la réparation du génome [Yura and Yshihma, Ann. Rev. Genet. (1979) 13:59-97]. Certains auteurs ont montré que les séquences des gènes *rpoB* et *rpoC* pouvaient être utilisées afin de construire des arbres phylogénétiques [Rowland et al. Biochem. Soc. Trans. (1992) 21 :40S] permettant de séparer les différents embranchements et sous-embranchements parmi les règnes du vivant.

Avant d'exposer plus en détail l'invention, différents termes, utilisés dans la description et les revendications, sont définis ci-après :
- Par « acide nucléique extrait de bactéries » on entend soit l'acide nucléique total, soit l'ADN génomique, soit les ARN messagers, soit encore l'ADN obtenu à partir de la transcription inverse des ARN messagers.
- Un « fragment nucléotidique » ou un « oligonucléotide » sont deux termes synonymes désignant un enchaînement de motifs nucléotidiques caractérisé par une séquence informationnelle des acides nucléiques naturels (ou éventuellement modifiés) et susceptibles de s'hybrider, comme les acides nucléiques naturels, avec un fragment nucléotidique complémentaire ou sensiblement complémentaire, dans des conditions prédéterminés de stringence stricte. L'enchaînement peut contenir des motifs nucléotidiques de structure différente de celle des acides nucléiques naturels. Un fragment nucléotidique (ou oligonucléotide) peut contenir par exemple jusqu'à 100 motifs nucléotidiques. Il contient généralement au moins 8, et en particulier au moins 12 motifs nucléotidiques, en particulier de 18 à 35, et peut être obtenu à partir d'une molécule d'acide nucléique naturelle et/ou par recombinaison génétique et/ou par synthèse chimique.
- Un motif nucléotidique est dérivé d'un monomère qui peut être un nucléotide naturel d'acide nucléique dont les éléments constitutifs sont un sucre, un groupement phosphate et une base azotée choisie parmi l'adénine (A), la guanine (G), l'uracile (U), la cytosine (C), la thymine (T) ; ou bien le monomère est un nucléotide modifié dans l'un au moins des trois éléments constitutifs précédents ; à titre d'exemple, la modification peut intervenir soit au niveau des bases, avec des bases modifiées telles que l'inosine, qui peut s'hydrider avec toute base A, T, U, C ou G, la méthyl-5-désoxycytidine, la désoxyuridine, la diméthylamino-5-désoxyuridine ou toute autre base modéfiée capable d'hybridation, soit au niveau du sucre, par exemple le ermplacement d'ua moins un désoxyribose par un polyamide [Nielsen PE et al., Science (1991) 254:1497-1500], soit encore au niveau du groupement phosphate, par exemple par remplacement par des esters choisis notamment parmi les diphosphates, les alkylphosphonates et les phosphorothioates.
- Par « hybridation », on entend le processus au cours duquel, dans des conditions appropriées, deux fragments nucléotidiques ayant des séquences suffisamment complémentaires sont susceptibles de s'associer par des liaisons hydrogène stables et spécifiques, pour former un double brin. Les conditions d'hybridation sont déterminées par la « stringence », c'est à dire la rigueur des conditions opératoires. L'hybridation est d'autant plus spécifique qu'elle est effectuée à plus forte stringence. La stringence est fonction notamment de la composition en bases d'un duplex sonde/cible, ainsi que par le degré de mésappariement entre deux acides nucléiques. La stringence peut également être fonction des paramètres de la réaction d'hybridation, tels que la concentration et le type d'espèces ioniques présentes dans la solution d'hybridation, la nature et la concentration d'agents dénaturants et/ou la température d'hybridation. La stringence des conditions dans lesquelles une réaction d 'hybridation doit être réalisée dépend notamment des sondes utilisées. Toutes ces données sont bien connues et les conditions appropriées peuvent éventuellement être déterminées dans chaque cas par des expériences de routine. En général, selon la longueur des sondes utilisées, la température pour la réaction d'hybridation est comprise entre environ 20 et 65°C, en particulier entre 35 et 65°C dans une solution saline à une concentration d'environ 0,8 à 1 M.
- Une « sonde » est un fragment nucléotidique possédant une spécificité d'hybridation dans des conditions déterminées pour former un complexe d 'hybridation avec un acide nucléique ayant, dans le cas présent, une séquence nucléotidique comprise soit dans un ARN messager, soit dans un ADN obtenu par transcription inverse dudit ARN messager, produit de transcription ; une sonde peut être utilisée à des fins de diagnostic (notamment sondes de capture ou de détection) ou à des fins de thérapie,
- Une « sonde de capture » est une sonde immobilisée ou immobilisable sur un support solide par tout moyen approprié, par exemple par covalence, par adsorption, ou par synthèse directe sur un solide. Des exemples de supports comprennent les plaques de microtitration et les puces à ADN.
- Une « sonde de détection » est une sonde marquée au moyen d'un agent marqueur choisi par exemple parmi les isotopes radioactifs, les enzymes, en particulier les enzymes susceptibles d'agir sur un substrat chromogéne, fluorigène ou luminescent (notamment une peroxydase ou une phosphatase alcaline), les composés chimiques chromophores, les composés chromogènes, fluorigènes ou luminescents, les analogues des bases nucléotidiques et les ligands tels que la biotine.
- Une « sonde d'espèce » est une sonde permettant l'identification spécifique de l'espèce d'une bactérie.
- Une « sonde de genre » est une sonde permettant l'identification spécifique du genre d'une bactérie.
- Une « amorce » est une sonde comprenant par exemple 10 à 100 motifs nucléotidiques et possédant une spécificité d'hybridation dans des conditions déterminées pour les réactions d'amplification enzymatique.
- Par « réaction d'amplification » on entend une réaction de polymérisation enzymatique, par exemple dans une technique d'amplification telle que la PCR, initiée par des oligonucléotides amorces et utilisant une ADN polymérase.
- Par « réaction de séquençage », on entend l'obtention de la séquence d'un fragment d'acide nucléique ou d'un gène complet par un procédé de polymérisation abortive à partir d'amorces oligonucléotidiques et utilisant lesdits didésoxynucléotides (Sanger F, Coulson AR (1975), J.Mol.Biol. 94 : 441) ou hybridations multiples avec des sondes multiples fixées sur support solide telles qu'utilisées dans les puces ADN par exemple.

Les séquences des gènes *rpo*B des bactéries *Streptococcus pneumoniae, Streptococcus pyogenes, Streptococcus mutans et Streptococcus agalactiae* ont été décrites dans la littérature.

Les inventeurs ont déterminé les séquences complètes des gènes *rpoB* d'autres espèces de bactéries du genre *Streptococcus* et apparentées: *Streptococcus anginosus* et *Streptococcus equinus,* d'*Abiotrophia defectiva*, et une très large portion du gène pour *Streptococcus mutans* et *Enterococcus faecalis*. Ces espèces ont été choisies par les inventeurs comme représentant les principaux groupes génétiques déterminés sur la base de l'étude du gène 16S dans les bactéries du genre *Streptococcus* et genres apparentés, encadrant l'ensemble des espèces actuellement décrites dans ce genre, de sorte que l'alignement des séquences *rpoB* obtenues chez ces espèces puisse encadrer vraisemblablement l'ensemble des séquences *rpoB* de toutes les espèces de ces genres bactériens plus précisément, il s'agit donc des espèces phylogénétiquement les plus divergentes parmi l'ensemble des espèces actuellement décrites dans ce genre, de sorte que l'alignement des séquences *rpob* obtenu chez ces espèces puisse encadrer phylogénétiquement vraisemblablement l'ensemble des séquences *rpob* de toutes les espèces de ce genre bactérien.

A partir de ces séquences complètes ou quasi complètes et après de nombreuses tentatives infructueuses tel que rapporté dans les exemples 1 et 2 ci-après, les inventeurs ont mis en évidence les séquences consensus et spécifiques SEQ.ID. n° 6 et 7 suivantes :
- SEQ ID N° 6 : 5'-AARYTNGGMCCTGAAGAAAT-3', et
- SEQ ID N°7: 5'-TGNARTTTRTCATCAACCATGTG-3',
dans lesquelles :
- N représente l'inosine ou l'un des 4 nucléotides A, T, C ou G,
- R représente A ou G,
- M représente A ou C, et
- Y représente C ou T,
et les séquences complémentaires.

Les inventeurs ont déterminé lesdites séquences SEQ.ID.n°6 et 7 comme étant non seulement consensuelles entre toutes les bactéries du genre *Streptococcus* et desdits 4 genres apparentés mais en outre spécifiques de la famille des bactéries du genre *Streptococcus* et desdits 4 genres apparentés.

A la position correspondant à un nucléotide N, Y, M ou R dans les séquences SEQ.ID. n°6 et 7 on trouve des nucléotides variables dans les séquences cibles complémentaires en fonction de l'espèce de la bactérie considérée, mais tous les autres nucléotides sont conservés dans toutes les espèces des bactéries du genre *Streptococcus* et desdits 4 genres apparentés.

Des séquences SEQ.ID n°6 et 7 ainsi définies sont présentes dans les gènes *rpoB* de toute bactérie du genre *Sreptococcus* et desdits 4 genres apparentés et spécifiques des bactéries du genre *Streptococcus* et desdits 4 genres apparentés et peuvent donc fournir des sondes de genre ou des amorces d'amplification pour détecter toute bactérie du genre *Streptococcus* et desdits 4 genres apparentés.

A cet effet, la présente invention a donc pour objet un oligonucléotide qui comprend une séquence d'au moins 8, de préférence au moins 12, de préférence encore de 18 à 35, motifs nucléotidiques, dont au moins une séquence de 8, de préférence 12, de préférence encore 18 motifs consécutifs inclus dans l'une des séquences SEQ.ID. n° 6 et 7 suivantes :
- SEQ ID N° 6 : 5'-AARYTNGGMCCTGAAGAAAT-3', et
- SEQ ID N°7: 5'-TGNARTTTRTCATCAACCATGTG-3',
dans lesquelles :
- N représente l'inosine ou l'un des 4 nucléotides A, T, C ou G,
- R représente A ou G,
- M représente A ou C, et
- Y représente C ou T, et les séquences complémentaires.

La présente invention a également pour objet un mélange d'oligonucléotides, **caractérisé en ce qu**'il est constitué d'un mélange équimolaire de d'oligonucléotides selon l'invention, ayant tous une séquence différente et comprenant tous une séquence incluse dans SEQ ID n°6 ou tous une séquence incluse dans SEQ ID n°7.

Plus particulièrement, la présente invention a également pour objet un mélange de dits oligonucléotides, constitué d'un mélange équimolaire de 32 oligonucléotides de séquences différentes comprenant chacune au moins 15, de préférence encore au moins 18 motifs nucléotidiques consécutifs inclus dans la séquence suivante :
- SEQ ID N° 6 : 5'-AARYTNGGMCCTGAAGAAAT-3',
dans laquelle :
- R représente A ou G,
- Y représente C ou T,
- M représente A ou C, et
- N représente A, T, C ou G,
et les séquences complémentaires.

La présente invention a également pour objet un mélange de dits oligonucléotides, constitué d'un mélange équimolaire de 8 oligonucléotides de séquences différentes comprenant chacune au moins 15, de préférence encore au moins 18 motifs nucléotidiques consécutifs inclus dans la séquence suivante :
- SEQ ID N° 6 : 5'-AARYTNGGMCCTGAAGAAAT-3',
dans laquelle :
- R représente A ou G,
- Y représente C ou T,
- M représente A ou C, et
- N représente l'inosine,
et les séquences complémentaires.

La présente invention a également pour objet un mélange de dits oligonucléotides, constitué d'un mélange équimolaire de 16 oligonucléotides de séquences différentes comprenant chacune au moins 15, de préférence encore au moins 21 motifs nucléotidiques consécutifs inclus dans la séquence suivante :
- SEQ ID N°7: 5'-TGNARTTTRTCATCAACCATGTG-3',
dans laquelle :
- R représente A ou G, et
- N représente A, T, C ou G, et les séquences complémentaires.

La présente invention a également pour objet un mélange de dits oligonucléotides, constitué d'un mélange équimolaire de 4 oligonucléotides de séquences différentes comprenant chacune au moins 15, de préférence encore au moins 21 motifs nucléotidiques·consécutifs inclus dans la séquence suivante :
- SEQ ID N°7: 5'-TGNARTTTRTCATCAACCATGTG-3',
dans laquelle :
- R représente A ou G, et
- N représente l'inosine,
et les séquences complémentaires.

Lesdits mélanges d'oligonucléotides peuvent s'hybrider avec une séquence complémentaire incluse dans le gène *rpoB* de toutes les bactéries du genre *Streptococcus* et desdits 4 genres apparentés et peuvent donc être utilisés à titre de sonde de genre ou amorces d'amplification pour la détection ou respectivement l'amplification d'un fragment de gène *rpo*B d'une dite bactérie.

Pour préparer un dit mélange équimolaire d'oligonucléotides selon les synthèses d'oligonucléotides connues de l'homme de l'art, il suffit de mettre en oeuvre un mélange équimolaire de 4 ou 2 nucléotides pour les nucléotides correspondant à N ou respectivement K, N, R ou Y, à savoir :
- un mélange équimolaire des 4 nucléotides, A, T, C et G pour les nucléotides correspondant à N dans lequel N représente A, T, C ou G, et
- un mélange équimolaire des 2 nucléotides T et G pour les nucléotides correspondant à K,
- un mélange équimolaire des 2 nucléotides A et C pour les nucléotides correspondant, à N,
- un mélange équimolaire des 2 nucléotides A et G pour les nucléotides correspondant à R, et
- un mélange équimolaire des 2 nucléotides C et T pour un nucléotide représenté par Y.

On obtient ainsi un mélange équimolaire de 32 (2³x4) et 16 (2²x4) nucléotides de séquences différentes pour respectivement les 2 séquences SEQ ID n°6 et 7.

Dans lesdits mélanges équimolaires d'oligonucléotides selon l'invention, du fait que « N » représente l'inosine qui peut s'hybrider avec toute base ou un mélange équimolaire des 4 bases A, T, C, G, les séquences SEQ.ID.n° 6 et 7 peuvent s'hybrider avec la séquence complémentaire incluse dans le gène *rpoB* de toutes les bactéries du genre *Streptococcus* et desdits 4 genres apparentés.

En outre, ces séquences consensus SEQ.ID. n° 6 et SED ID n° 7 encadrent des séquences hyper variables dont la séquence est spécifique pour chaque espèce de bactérie du genre *Streptococcus.* Ces séquences encadrées par SEQ.ID. n° 6 et 7 peuvent donc être utilisées à titre de sonde d'espèce des bactéries du genre *Streptococcus* et desdits 4 genres apparentés.

De plus, les séquences SEQ.ID. n°6 et 7 ont été déterminées comme encadrant un fragment du gène *rpob* comprenant une zone dont la longueur variable est d'environ 720 pb et comme comprenant les plus courtes séquences spécifiques pour chaque espèce de la bactérie du genre *Streptococcus* et desdits 4 genres apparentés.

Les inventeurs ont ainsi pu mettre en évidence des sondes d'espèce pour chacune des 28 espèces de bactérie du genre *Streptococcus* et desdits 4 genres apparentés étudiées correspondant aux séquences SEQ.ID.n° 8 à 35 décrites à l'exemple 2 ci-après, encadrées par les séquences consensus SEQ.ID.n° 6 et 7.

Dans les séquences SEQ.ID n° 1 à 3 et 5 décrites dans le listage de séquences en fin de description :
- le nucléotide M représente A ou C,
- le nucléotide K représente T ou G,
- le nucléotide R représente A ou G,
- le nucléotide W représente A ou T,
- le nucléotide Y représente C ou T,
- le nucléotide N représente A, T, C, G ou I,
- le nucléotide S représente C ou G,
- le nucléotide V représente A, C ou G.

Les séquences consensus tirées de SEQ.ID.n° 6 et 7 mises en évidence selon la présente invention, peuvent être utilisées à titre de sonde de genre, d'amorce d'amplification ou de réaction de séquençage dans des procédés de détection de bactérie du genre *Streptococcus* et desdits 4 genres apparentés par identification moléculaire.

Les séquences tirées des séquences SEQ.ID.n° 6 et 7 permettent dont non seulement de préparer des sondes de genre des bactéries du genre *Streptococcus* et desdits 4 genres apparentés mais aussi de détecter et identifier l'espèce de ladite bactérie par amplification et séquençage en utilisant lesdites séquences comme amorces.

La séquence complète du gène *rpoB* peut être utilisée pour identifier la bactérie pas seulement par l'étude de sa séquence primaire, mais aussi, par l'étude des structures secondaire et tertiaire de l'ARN messager provenant de la transcription de la séquence complète d'ADN.

Un autre objet de la présente invention est un oligonucléotide ou un fragment de gène *rpo*B ayant une séquence consistant dans les séquences SEQ.ID.n°8 à 35, y compris donc les séquences SEQ ID n°11, 12, 14 et 22 des bactéries *Streptococcus pyogenes, Streptococcus pneumoniae, Streptococcus mutans* et respectivement *Streptococcus agalactiae* et parmi les oligonucléotides ou fragments de séquences complémentaires tels que définis ci-dessus.

Les inventeurs, après analyse des différentes séquences, ont déterminé par comparaison deux à deux de toutes les séquences SEQ ID n°8 à 35, que, le taux d'homologie entre deux séquences différentes parmi lesdites séquences SEQ ID n°8 à 35 est au maximum de 98,7%. En dessous de 98,7% d'homologie entre les séquences, celles-ci identifient des bactéries d'espèces différentes.

Les oligonucléotides, fragments de gène et gènes objets de la présente invention, ont été décrits comme comportant des séquences d'ADN, c'est-à-dire avec des oligonucléotides A, T, C et G. Toutefois, la présente invention a également pour objet des oligonucléotides comprenant des séquences d'ARN correspondantes, c'est-à-dire dans lesquelles T est remplacé par U.

Dans la présente description, on entend par "séquences inverses et séquences complémentaires", les séquences suivantes :
- la séquence inverse de ladite séquence,
- la séquence complémentaire de ladite séquence, et
- la séquence complémentaire de la séquence inverse de ladite séquence.

Les séquences SEQ.I. n°1 à 35 peuvent être préparées par génie génétique et/ou par synthèse chimique, notamment par synthèse automatique, en utilisant les techniques bien connues de l'homme du métier.

Une première application d'un oligonucléotide selon l'invention est son utilisation comme sonde pour la détection, dans un échantillon biologique, de bactéries de l'une des espèces du genre *Streptococcus* et desdits 4 genres apparentés qui comprend une séquence nucléotidique dans l'une des séquences SEQ.ID. n°6 à 35, et leurs séquences complémentaires.

Un oligonucléotide comprenant les séquences SEQ.ID.n° 6 et 7 sera utilisé à titre de sonde de genre et un oligonucléotide comprenant une séquence comprise dans ou comprenant l'une des séquences SEQ.ID. n° 8 à 35, sera utilisée à titre de sonde d'espèce.

Plus particulièrement, la présente invention a pour objet un oligonucléotide consistant en une séquence spécifique d'une espèce d'une bactérie du genre *Streptococcus* et dits genres apparentés, de 20 à 100 nucléotides consécutifs, de préférence encore au moins 30 nucléotides consécutifs inclus dans l'une des dites séquences SEQ ID n°8 à 35,ou le cas échéant un mélange équimolaire de dits oligonucléotides de séquences différentes.

De préférence, lesdites séquences comprises dans l'une des séquences SEQ ID n°8 à 35, ayant de préférence au moins 20 nucléotides, de préférence encore au moins 30 nucléotides consécutifs inclus dans l'une des séquences SEQ ID n°8 à 35, constituent les séquences spécifiques des différentes espèces respectives les plus courtes, utilisables comme sonde d'espèces des bactéries *Streptococcus* et des dits 4 genres apparentés concernées.

Les sondes selon l'invention peuvent être utilisées, à des fins de diagnostic, comme mentionné précédemment, par la détermination de la formation ou de l'absence de formation d'un complexe d'hybridation entre la sonde et un acide nucléique cible dans un échantillon, selon toutes les techniques d'hybridation connues et notamment les techniques de dépôt ponctuel sur filtre, ditres « DOT-BLOT » [Maniatis et al. (1982) Molecular Cloning, Cold Spring Harbor], les techniques de transfert d'ADN dites « SOUTHERN BLOT » [Southern E.M., J. Mol. Biol. (1975) 98:503], les techniques de transfert d'ARN dites « NOTHERN BLOT », ou les techniques dites « sandwich », en particulier avec une sonde de capture et/ou une sonde de détection, lesdites sondes étant capables de s'hybrider avec deux régions différentes de l'acide nucléique cible, et l'une au moins desdites sondes (généralement la sonde de détection étant capable de s'hybrider avec une région de la cible qui est spécifique de l'espèce, étant entendu que la sonde de capture et la sonde de détection doivent avoir des séquences nucléotidiques au moins partiellement différentes.

L'acide nucléique à détecter (cible) peut être de l'ADN ou de l'ARN (le premier obtenu après amplification par PCR). Dans le cas de la détection d'une cible de type acide nucléique double brin, il convient de procéder à la dénaturation de ce dernier avant la mise en oeuvre du procédé de détection. L'acide nucléique cible peut être obtenu par extraction selon les méthodes connues des acides nucléiques d'un échantillon à examiner. La dénaturation d'un acide nucléique double brin peut être effectuée par les méthodes connues de dénaturation chimique, physique ou enzymatique, et en particulier par chauffage à une température appropriée, supérieure à 80°C.

Pour mettre en oeuvre les technique d'hybridation précitées, et en particulier les techniques « sandwich », une sonde de l'invention, appelée sonde de capture est immobilisée sur un support solide, et une autre sonde de l'invention, appelée sonde de détection, est marquée avec un agent marqueur. Les exemples de support et d'agent marqueur sont tels que définis précédemment.

De manière avantageuse, une sonde d'espèce est immobilisée sur un support solide, et une autre sonde d'espèce est marquée par un agent marqueur.

Une autre application d'un oligonucléotide de l'invention est son utilisation comme amorce nucléotidique comprenant un oligonucléotide monocaténaire choisi parmi les oligonucléotides ayant une séquence d'au moins 12 motifs nucléotidiques inclus dans l'une des séquences SEQ.ID. n° 6 à 35, qui est utilisable dans la synthèse d'un acide nucléique en présence d'une polymérase par un procédé connu en soi, notamment dans des méthodes d'amplification utilisant une telle synthèse en présence d'une polymérase (PCR, RT-PCR, etc.). En particulier, une amorce de l'invention peut être utilisée pour la transcription inverse spécifique d'une séquence d'ARN messager de bactérie d'une espèce du genre *Streptococcus* et desdits 4 genres apparentés pour obtenir une séquence d'ADN complémentaire correspondante. Une telle transcription inverse peut constituer le premier stade de la technique RT-PCR, le stade suivant étant l'amplification par PCR de l'ADN complémentaire obtenu. On peut également utiliser les amorces de l'invention pour l'amplification spécifique par réaction de polymérisation en chaîne de la séquence totale de l'ADN du gène *rpoB* d'une espèce du genre *Streptococcus* et desdits 4 genres apparentés.

Selon un cas particulier ladite amorce comprenant un oligonucléotide de l'invention comprend en outre la séquence sens ou anti-sens d'un promoteur reconnu par une ARN polymérase (promoteurs T7, T3, SP6 par exemple [Studier FW, BA Moffatt (1986) J. Mol. Biol. 189 :113] : de telles amorces sont utilisables dans des procédés d'amplification d'acide nucléique faisant intervenir une étape de transcription, tels que, par exemple, les techniques NASBA ou 3SR [Van Gemen B. et al. Abstract MA 1091, 7th International Conference on AIDS (1991) Florence, Italy].

Une amorce nucléotidique comprenant un oligonucélotide choisi parmi les oligonucléotides ayant une séquence comprenant l'une des séquences SEQ ID n° 6 à 35 ou une séquence incluse dans SEQ.ID. n° 6 à 35, est utilisable pour le séquençage total ou partiel du gène *rpoB* d'une souche quelconque d'une espèce du genre *Streptococcus* et desdits 4 genres apparentés.

Le séquençage du gène *rpoB* partiel ou complet chez toute bactérie du genre *Streptococcus* et genres apparentés permet l'identification de toute bactérie *Streptococcus* et desdits 4 genres apparentés par analyse bio informatique de cette séquence et la reconnaissance de nouvelles espèces de bactéries *Streptococcus* et desdits 4 genres apparentés inconnues.

De préférence, dans une utilisation comme amorce ou pour le séquençage des gènes *rpoB,* on utilise un dit mélange d'oligonucléotides selon l'invention, et de préférence encore des dits mélanges d'oligonucléotides consistant dans les séquences SEQ ID n°6 et SEQ ID n°7.

Plus précisément, la présente invention fournit un procédé de détection par identification d'une bactérie de l'une des espèces du genre *Streptococcus* et desdits 4 genres apparentés caractérisé en ce qu'on utilise :
- un dit fragment dudit gène *rpoB* de ladite bactérie selon la présente invention, consistant en une séquence nucléotidique choisie parmi l'une des séquences SEQ.ID.n° 8 à 35, les séquences complémentaires, utile notamment comme sonde d'espèces, et/ou
- un dit oligonucléotide selon la présente invention consistant en une dite séquence spécifique incluse dans l'une des séquences SEQ ID n°8 à 35, les séquences complémentaires, utile notamment comme sonde d'espèces, et/ou
- un oligonucléotide ou dit mélange d'oligonucléotides selon la présente invention comprenant une séquence constituée de motifs nucléotidiques consécutifs, inclus dans l'une des séquences SEQ.ID.n°6 et 7, utile notamment comme sonde de genre ou .amorce d'amplification.

De préférence, dans ledit procédé de détection selon l'invention, on utilise au moins un dit mélange d'oligonucléotides selon la présente invention, dont les séquences consistent dans les séquences SEQ ID n° 6 et 7, et leurs séquences inverses et séquences complémentaires dans lesquelles de préférence encore N représente l'inosine.

Dans un premier mode de réalisation d'un procédé de détection selon l'invention, on cherche à mettre en évidence la présence d'une bactérie du genre *Streptococcus* et desdits 4 genres apparentés, on réalise les étapes dans lesquelles :
1. on met en contact au moins une sonde de genre comprenant un dit mélange d'oligonucléotides de séquences comprenant ou comprises dans l'une des séquences SEQ.ID.n° 6 et 7, les séquences complémentaires selon l'invention, avec un échantillon contenant ou susceptible de contenir des acides nucléiques d'au moins une telle bactérie du genre *Streptococcus* et desdits 4 genres apparentés, et
2. on détermine la formation ou l'absence de formation d'un complexe d'hybridation entre ladite sonde de genre et les acides nucléiques de l'échantillon, et on détermine la présence d'une dite bactérie du genre *Streptococcus* et desdits 4 genres apparentés s'il y a formation d'un complexe d'hybridation.

Dans une deuxième mode de réalisation d'un procédé de détection d'une bactérie du genre *Streptococcus* et desdits 4 genres apparentés, on réalise les étapes dans lesquelles :
1. On met en contact des amorces d'amplification comprenant desdits mélanges d'oligonucléotides comprenant une séquence incluse dans lesdites séquences SEQ.ID. n° 6 et 7 et séquences complémentaires selon l'invention, avec un échantillon contenant ou susceptibles de contenir des acides nucléiques d'au moins une telle bactérie du genre *Streptococcus* et desdits 4 genres apparentés avec :
   - comme amorce 5' : un dit mélange d'oligonucléotides comprenant une séquence incluse dans la séquence SEQ.ID.n° 6, ou de préférence consistant dans ladite séquence SEQ ID n° 6 complète, ou une séquence complémentaire selon l'invention ;
   - comme amorce 3' : un dit mélange d'oligonucléotides comprenant une séquence incluse dans la séquence la séquence SEQ.ID.n° 7 ou de préférence consistant dans ladite séquence SEQ ID n° 7 complète, ou respectivement une séquence complémentaire selon l'invention.
2. On réalise une amplification d'acides nucléiques par réaction de polymérisation enzymatique et on détermine l'apparition ou l'absence d'un produit d'amplification, et on détermine ainsi la présence d'une dite bactérie dans l'échantillon si un produit d'amplification est apparu.

Ce deuxième mode de réalisation peut être utilisé pour détecter spécifiquement le genre d'une bactérie du genre *streptococcus* ou desdits 4 genres apparentés.

Mais, à l'étape 2 de ce deuxième mode de réalisation, on peut chercher à détecter spécifiquement une espèce donnée d'une bactérie du genre *Streptococcus* choisie parmi les espèces *Streptococcus mutans, Streptococcus oralis, Streptococcus pneumoniae, Streptococcus pyogenes, Streptococcus salivarius, Streptococcus sanguinis, Streptococcus suis, Streptococcus acidominimus, Streptococcus agalactiae, Streptococcus anginosus, Streptococcus constellatus, Streptococcus difficilis, Streptococcus dysgalactiae, Streptococcus equi, Streptococcus equinus, Streptococcus intermedius, Streptococcus mitis, Streptococcus bovis, Granulicatella adjacens, Abiotrophia defectiva, Enterococcus avium, Enterococcus casselliflavus, Enterococcus faecalis, Enterococcus faecium, Enterococcus gallinarum, Enterococcus sacharolyticus, Gemella haemolysans et Gemella morbillorum.,* comme décrit dans la variante de réalisation d'un procédé de détection spécifique d'une espèce de dites bactéries, décrite ci-après

Comme cela a été précédemment exposé en introduction, les genres *Streptococcus, Enterococcus, Granulicatella, Abiotrophia,* et *Gemella* comportent plus d'espèces bactériennes que celles qui ont été effectivement séquencées dans ce travail. Toutefois, les espèces séquencées ont été choisies telles qu'elles encadrent toutes les espèces connues dans ces genres bactériens et sont en nombre suffisant pour démontrer l'application de la séquence *rpoB* à l'identification des espèces de ces genres.

Dans une variante de réalisation d'un procédé de détection spécifique d'une espèce desdites bactéries selon l'invention, on réalise les étapes dans lesquelles :
1- on met en contact un échantillon contenant ou susceptible de contenir des acides nucléiques d'au moins une telle bactérie, avec au moins une sonde d'espèce consistant dans un dit fragment de gène ou dit oligonucléotide comprenant une séquence incluse dans l'une des séquences SEQ.ID n° 8 à 35, de préférence un oligonucléotide consistant dans l'une desdites séquences SEQ.ID. n° 8 à 35, les séquences complémentaires selon l'invention, et
2- on détermine la formation ou l'absence d'un complexe d'hybridation entre ladite sonde et les acides nucléiques de l'échantillon, et on détermine ainsi la présence de ladite bactérie dans l'échantillon s'il y a formation d'un complexe d'hybridation.

Dans une autre variante de réalisation du procédé selon l'invention dans lequel on cherche à détecter spécifiquement une espèce donnée d'une bactérie du genre *Streptococcus* et desdits 4 genres apparentés choisie parmi les 28 espèces citées ci-dessus, le procédé comprend les étapes dans lesquelles, dans un échantillon contenant ou susceptible de contenir des acides nucléiques d'au moins une dite bactérie :
a) on réalise une réaction de séquençage d'un fragment du gène *rpoB* amplifié d'une dite bactérie donnée à l'aide des amorces nucléotidiques consistant dans desdits mélanges d'oligonucléotides comprenant des séquences incluses dans la séquence SEQ.ID. n° 6 comme amorce 5', et SEQ.ID. n° 7 comme amorce 3', de préférence les séquences consistant dans lesdites séquences SEQ.ID. n° 6 et 7, et leurs séquences complémentaires, et
b) on détermine la présence ou l'absence de l'espèce donnée de ladite bactérie en comparant la séquence dudit fragment obtenu avec la séquence du gène complet *rpoB* de ladite bactérie ou la séquence d'un fragment du gène *rpoB* de ladite bactérie comprenant lesdites séquences n° 8 à 35 et séquences complémentaires selon l'invention, et on détermine ainsi la présence de ladite bactérie dans l'échantillon si la séquence du fragment obtenue est identique à la séquence connue du genre ou du fragment de gène *rpoB* de ladite bactérie.

La présente invention a également pour objet une trousse de diagnostic utile dans un procédé selon l'invention comprenant au moins un dit fragment de gène ou dit oligonucléotide de séquence comprise ou consistant dans les séquences SEQ.ID. n° 8 à 35 ou un dit oligonucléotide ou mélange d'oligonucléotides comprenant une séquence incluse dans une des séquences SEQ.ID. n° 6 et 7, et/ou au moins un dit fragment de gène *rpoB* d'une dite bactérie comprenant les séquences SEQ.ID. n° 8 à 35, et les séquences complémentaires selon l'invention.

Avantageusement, un trousse selon la présente invention comporte des dits oligonucléotides sous forme de "biopuces", c'est-à-dire fixés sur des supports solides, notamment en verre, selon le procédé décrit dans le brevet US 5 744 305 (Affymetrix, Fodor et al) en utilisant la stratégie de reséquençage décrite dans la demande WO 95/11995 (Affymax, Chee et al) ou selon la méthode décrite par A Troesch et al. dans J. Clin. Microbiol., vol. 37(1), p 49-55, 1999. Les oligonucléotides synthétisés sur la "biopuce" réalisent le reséquençage de la région hyper variable du gène rpoB. Ce procédé présente un avantage considérable en terme de coût de production et sans compromis sur la qualité de l'identification des différentes espèces de part le choix de ces séquences d'identification. De préférence, ces oligonucléotides fixés sur le support solide de la "biopuce" comportent de 10 à 30 bases par exemple 20 bases, avec une position d'interrogation située dans la région centrale comme par exemple en 12ème position par rapport à l'extrémité 3' de la séquence pour des oligonucléotides de 20 bases. Un autre exemple consiste à utiliser des oligonucléotides de 17 bases, avec 2 positions d'interrogations : une en 10ème et une en 8ème position. D'autres oligonucléotides ont des longueurs comprises entre 10 et 25 nucléotides. Les positions d'interrogation varient alors en fonction de la longueur de l'oligonucléotide.

L'analyse est effectuée sur le système complet GeneChip® (référence 900228, Affymetrix, Santa Clara, CA) qui comprend le lecteur GeneArray®, le four d'hybridation GeneChip®, la station fluidique GeneChip® et le logicel d'analyse GeneChip®.

Un oligonucléotide selon l'invention peut aussi être utilisé à titre de sonde de thérapie génique pour traiter les infections provoquées par une souche appartenant à une espèce du genre *Streptococcus* et desdits 4 genres apparentés, ladite sonde comprenant un oligonucléotide tel que défini précédemment. Cette sonde de thérapie génique, capable de s'hybrider sur l'ARN messager et/ou sur l'ADN génomique desdites bactéries, peut bloquer les phénomènes de traduction et/ou transcription et/ou de réplication.

Le principe des méthodes de thérapie génique est connu et repose notamment sur l'utilisation d'une sonde correspondant à un brin anti-sens : la formation d'un hybride entre la sonde et le brin sens est capable de perturber au moins l'une des étapes du décryptage de l'information génétique. Les sondes de thérapie génique sont donc utilisables comme médicaments antibactériens, permettant de lutter contre les infections causées par les bactéries des espèces du genre *Streptococcus* et desdits 4 genres apparentés.

L'invention sera mieux comprise à l'aide de l'exposé ci-après, divisé en exemples, qui concernent des expériences effectuées dans le but de réaliser l'invention et qui sont données à titre purement illustratif.

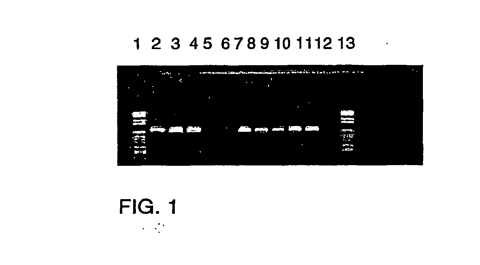

La figure 1 représente la visualisation des produits d'amplification par coloration au bromure d'éthidium après électrophorèse sur un gel d'agarose obtenu à l'exemple 3.

### Exemple 1. Séquence du gène rpoB de trois espèces du genre Streptococcus et genre apparenté: Abiotrophia defectiva, Streptococcus anginosus et Streptococcus equinus.

La séquence complète du gène *rpoB* des bactéries des espèces *Abiotrophia defectiva, Streptococcus anginosus* et *Streptococcus equinus* a été déterminée par amplification enzymatique et séquençage automatique disponible chez les Streptocoques. Le choix de ces espèces a été basé sur l'analyse de l'arbre 16S qui montre une divergence génétique couvrant l'ensemble de l'arbre phylogénétique des streptocoques.

### Stratégie et Séquençage :

Plusieurs séquences partielles de 510 pb de gènes *rpo-B* sont disponibles sur GenBank pour les 10 espèces de streptocoques suivantes : *Streptococcus intermedius, Streptococcus sanguinis, Streptococcus pneumoniae, Streptococcus parasanguinis, Streptococcus oralis, Streptococcus mitis, Streptococcus cristalus, Streptococcus constallatus, Streptococcus anginosus* et *Granulicatella adjacens.* [Majewski,J., Zawadzki,P., Pickerill,P., Cohan,F.M. and Dowson,C.G. Barriers to genetic exchange between bacterial species: Streptococcus pneumoniae transformation.J. Bacteriol. 182, 1016-1023 (2000)], mais les amorces utilisées par ces auteurs n'amplifient qu'une fraction des espèces du genre *Streptococcus* et il n'a donc pas été possible de mener à bien notre travail sur la base de ces seules données. II a donc fallu déterminer des amorces capables d'amplifier l'ensemble des souches de streptocoques, enterocoques, *Abiotrophia, Gemella, et Granulicatella.* Ces amorces devaient en outre encadrer une région présentant une diversité génétique suffisante pour permettre de distinguer deux espèces entre elles. Cependant, l'alignement de ces séquences partielles publiées a permis de déterminer les amorces communes suivantes : (la numération se réfère à la séquence complète du *Streptococcus. pyogenes*)
   SEQ ID N° 36 : 5'- AGACGGACCTTCTATGGAAAA -3' (amorce 748F),
   - SEQ.ID.N° 37 : 5'- GGACACATACGACCATAGTG -3' (amorce 116R), et
   SIQ N° 38 : 5'- GTTGTAACCTTCCCAWGTCAT -3' (amorce 830R).

Ces amorces ont permis de séquencer la partie centrale du gène rpoB de 714pb pour les cinq espèces choisies (*Streptococcus equinus, Streptococcus mutans, Streptococcus anginosus, Enterococcus faecalis, et Abiotrophia defectiva* A partir de ce fragment central, le séquençage a été poursuivi par la technique dite du génome Walker.

En dehors de cette zone publiée [Majewski,J., et al. J. Bacteriol. 2002, 182, 1016-1023], l'alignement des deux séquences complètes disponibles dans GenBank (*Streptococcus pneumoniae* [GenBank numéro d'accès AE008542] et *Streptococcus pyogenes* [GenBank numéro d'accès AE006480*)* ont permis de choisir les amorces suivantes :
- SEQ ID N° 39 : 5'- GTCTTCWTGGGYGATTTCCC -3' (amorce 2215R),
- SEQ ID N° 40 : 5'- ACCGTGGIGCWTGGTTRGAAT -3' (amorce 2057R),
- SEQ ID N° 41 : 5'- AACCAATTCCGYATYGGTYT -3'(amorce 1252R),
- SEQ ID N° 42 : 5'- AGIGGGTTTAACATGATGTC -3'(amorce 371 F),
- SEQ ID N° 43 : 5'- AGIGCCCAAACCTCCATCTC -3'(amorce 730F), et
- SEQ ID N° 44 : 5'- CTCCAAGTGAACAGATGTGTA -3'(amorce 585R).
Ces amorces ont permis d'étendre la région séquencée pour certaines des cinq souches choisies. De façon tout à fait inattendue, *E. faecalis* n'est pas amplifiée par ces amorces ; mais on a observé que la zone partielle séquencée présentait une homologie avec le gène *rpoB* de *Listeria monocytogenes,* c'est à dire avec une bactérie appartenant à un genre bactérien différent ce qui ne pouvait absolument pas être déduit des données existantes, on a donc choisi des amorces dans le gène *rpoB* de *Listeria* pour amplifier le gène *rpoB* de *Enterococcus faecalis.*
- SEQ ID N°45 : 5'- TTACCAAACTTAATTGAGATTCAAAC- 3' (amorce 180F)
- SEQ ID N°46 : 5'- AGTATTTATGGGTGATTTCCCA- 3' (amorce 410F)
- SEQ ID N°47 : 5'- GGACGTTATAAAATCAACAAAAAATT- 3' (amorce 910F)
- SEQ ID N°48 : 5'- AGTTATAACCATCCCAAGTCATG- 3' (amorce 2430R)
- SEQ ID N°49 : 5'- TGAAGTTTATCATCAACCATGTG- 3' (amorce 3280R)
- SEQ ID N°50 : 5'- CCCAAAACGTTGTCCACC- 3' (amorce 3360R)

Les séquences partielles ainsi obtenues pour les cinq souches choisies *(Streptococcus equinus, Streptococcus mutans, Streptococcus anginosus, Enterococcus faecalis, Abiotrophia defectiva*) ont permis de choisir les amorces suivantes :
- SEQ ID N°51 : 5'- AACCAAGCYCGGTTAGGRAT -3' (amorce 520R)
- SEQ ID N°52 : 5'- ATGTTGAACCCACTIGGGGTGCCAT -3' (amorce 2881 F) pour le séquençage des zones C- et N- terminales par Génome Walker.

Le séquençage est alors complet, comme en témoignent la détermination de la région codante, et l'alignement des protéines traduites des séquences nucléotidiques avec les deux protéines RpoB publiées de *Streptococcus pneumoniae* et *Streptococcus pyogenes*.

Plusieurs amorces consensus potentielles ont fait l'objet d'investigations pour obtenir un fragment susceptible de conduire à la séquence complète des gènes *rpoB* par élongations successives à partir d'une série d'amorces spécifiques.

Dans chacune des étapes ci-dessus, un grand nombre de tentatives avec des amorces théoriquement ou potentiellement appropriées ont échoue avant de déterminer les amorces mentionnées ci-dessus pour permettre d'amplifier et de séquencer par étapes successives la totalité des gènes *rpob* décrite ci-après.

Les réactions de séquençage ont été réalisées en utilisant les réactifs du kit ABI Prism dRhodamine Dye Terminator Cycle Sequencing Ready Reaction Kit (Perkin Elmer Applied Biosystems) selon les recommandations du fournisseur suivant le programme suivant : 30 cycles comprenant une étape de dénaturation à 94°C pendant 10 sec., une étape d'hybridation de l'amorce à 50°C pendant 10 sec. et une étape d'extension à 60°C pendant 2 minutes. Les produits de séquençage ont été séparés par électrophorèse sur un gel de polyacrylamide sur un séquenceur 377 DNA Sequencer (Perkin) et analysés pour former des séquences consensus par le logicel Sequence Assembler (Applied Biosystems).

Cette approche nous a permis de déterminer la séquence complète du gène *rpoB* chez deux espèces du genre *Streptococcus* et chez *Abiotrophia defectiva* :
SEQ.ID. n°1 : Séquence du gène *rpoB* de *Streptococcus anginosus.* Cette séquence mesure 4 523 paires de bases , possède un contenu en cytosine plus guanosine de 41 % et est déposée dans GenBank sous le numéro d'accession AF 535183 :
SEQ ID N°2 : Séquence du gène *rpoB* de *Streptococcus equinus.* Cette séquence mesure 4 118 paires de bases et possède un contenu en cytosine plus guanosine de 41 % est déposée dans GenBank sous le numéro GenBank accession AF 535187:
SEQ ID N°3 : Séquence du gène *rpoB* d'*Abiotrophia defectiva.* Cette séquence mesure 4 325 paires de bases, possède un contenu en cytosine plus guanosine de 47 %, et est déposée dans GenBank sous le numéro AF 535173 :
SEQ ID N°4 : Séquence partielle du gène *rpoB* de *Streptococcus mutans.* Cette séquence mesure 3198 paires de bases, elle possède un contenu en cytosine plus guanosine de 42 %, et est déposée dans GenBank sous le numéro AF 535167.
SEQ ID N°5 : Séquence partielle du gène *rpoB* d'*Enterococcus faecalis.* Cette séquence mesure 3096 paires de bases, elle possède un contenu en cytosine plus guanosine de 42 %, et est déposée dans GenBank sous le numéro AF 535175

Dans les séquences qui précèdent, le nucléotide K désigne T ou G, le nucléotide M désigne A ou C, le nucléotide R désigne A ou G, le nucléotide W désigne A ou T, le nucléotide Y désigne C ou T et le nucléotide N désigne A, T, C ou G.

### Exemple 2: Séquençage partiel du gène rpoB de 28 espèces du genre Streptococcus et genres apparentés.

A partir de l'alignement des séquences complètes du gènes *rpoB* chez *Streptococcus* spp. et *Abiotrophia defectiva* de l'exemple 1 et celles connues dans GenBank, (*Streptococcus pneumonia* AE008542 et *Streptococcus pyogenes* AE006480 ) un jeu d'amorces a été choisi pour l'amplification et le séquençage d'un fragment 709 à 740 pb de ce gène chez 28 souches type de ces genres bactériennes. Ces amorces ont pour séquence :
- SEQ ID N°6: 5'-AARYTIGMCCTGAAGAAAT-3'
- SEQ ID N°7: 5'-TGIARTTTRTCATCAACCATGTG-3'

La séquence SEQ ID n° 7 est utilisée à titre d'amorce 3' et représente donc la séquence inverse complémentaire du brin direct représenté dans les séquences SEQ ID n° 1 à 5 qui précèdent.

Ces amorces sont incorporées avec l'ADN extrait des bactéries dans une PCR selon les conditions suivantes : dénaturation à 95°C pendant 1 min suivie de 35 cycles comportant une étape de dénaturation à 94°C pendant 10 sec, une étape d'hybridation à 52°C pendant 10 sec et une étape d'élongation à 72°C pendant 30 sec.

Les produits amplifiés sont séquencés par les mêmes amorces SEQ ID N°6 et SEQ ID N°7 selon les conditions suivantes : dénaturation à 95°C pendant 1 min suivie de 30 cycles comportant une étape de dénaturation à 95°C pendant 30 sec, une étape d'hybridation à 52°C pendant 30 sec et une étape d'hybridation à 62°C pendant 1 min. Les produits de séquençage sont analysés par un séquenceur ABI PRISM 3100.

Les inventeurs ont déterminé la position de ces deux amorces SEQ.ID. n° 6 et SEQ.ID. n° 7, de façon à respecter les critères suivants :
1- séquence encadrée par ces deux amorces spécifiques de l'espèce de la bactérie. Cette condition est vérifiée après alignement des fragments de environ 720 pb avec l'ensemble des séquences des gènes bactériens *rpoB* disponibles dans les banques de données informatiques.
2- recherche d'une région d'identification la plus courte possible afin d'augmenter le plus possible la sensibilité de la détection moléculaire
3- la longueur des amorces de 18 à 22 pb,
4- séquence des amorces présentant une température de fusion voisine,
5- séquence des amorces ne permettant pas d'auto-hybridation ni de complémentarité.

Les fragments obtenus du gène *rpoB* des bactéries des espèces du genre *Streptococcus* et desdits genres apparentés ont environ 720 (709 A 732) paires de bases et leur séquence est spécifique de chaque espèce de ce genre et permettant donc l'identification moléculaire des bactéries des 28 espèces testées sont :
SEQ.ID. n°8 : séquence partielle du gène *rpoB* chez *Streptococcus suis* CIP 1032 17^{T} mesurant 709 paires de bases :
SEQ.ID.n°9 : séquence partielle du gène *rpoB Streptococcus sanguinis* CIP 55.128^{T} mesurant 725 paires de bases :
SEQ.ID. n°10 : séquence partielle du gène *rpoB Streptococcus salivarius* CIP 102503^{T} mesurant 728 paires de bases :
SEQ.ID. n°11 : séquence partielle du gène *rpoB* chez *Streptococcus pyogenes*
CIP 56.41^{T} mesurant 725 paires de bases :
SEQ.ID. n°12: séquence partielle du gène *rpoB* chez *Streptococcus pneumoniae* CIP 102911^{T} mesurant 724 paires de bases :
SEQ.ID. n°13 : séquence partielle du gène *rpoB* chez *Streptococcus oralis* CIP 102922^{T} mesurant 694 paires de bases :
SEQ.ID. n°14 : séquence partielle du gène *rpoB* chez *Streptococcus mutans* CIP 103220^{T} mesurant 728 paires de bases :
SEQ.ID.n°15 : séquence partielle du gène *rpoB* chez *Streptococcus mitis* CIP 103335^{T} mesurant 730 paires de bases :
SEQ.ID. n°16 : séquence partielle du gène *rpoB* chez *Streptococcus equinus* CIP 102504^{T} mesurant 697 paires de bases :
SEQ.ID. n°17 : séquence partielle du gène *rpoB* chez *Streptococcus constellatus*
CIP 103247^{T} mesurant 731 paires de bases :
SEQ.ID. n°18 : séquence partielle du gène *rpoB* chez *Streptococcus anginosus* CIP 102921^{T} mesurant 697 paires de bases :
SEQ.ID. n°19: séquence partielle du gène *rpoB* chez *Streptococcus dysgalactiae* CIP 102914^{T} mesurant 728 paires de bases :
SEQ.ID. n°20 : séquence partielle du gène *rpoB* chez *Streptococcus bovis* CIP 102302^{T} mesurant 728 paires de bases :
SEQ.ID. n°21: séquence partielle du gène *rpoB* chez *Streptococcus acidominimus* CIP 82.4^{T} mesurant 728 paires de bases :
SEQ.ID, n°22 : séquence partielle du gène *rpoB* chez *Streptococcus agalactiae*
CIP 103227^{T} mesurant 733 paires de bases :
SEQ.ID. n°23 : séquence partielle du gène *rpoB* chez *Streptococcus difficilis* CIP 103768^{T} mesurant 714 paires de bases :
SEQ.ID. n°24 : séquence partielle du gène *rpoB* chez *Streptococcus intermedius*
CIP 103248^{T} mesurant 728 paires de bases :
SEQ.ID. n°25 : séquence partielle du gène *rpoB* chez *Sreptococcus equi* CIP 102910^{T} mesurant 728 paires de bases
SEQ.ID. n°26 : séquence partielle du gène *rpoB* chez *Enterococcus gallinarum* CIP 103013^{T} mesurant 694 paires de bases :
SEQ.ID. n°27: séquence partielle du gène *rpoB* chez *Enterococcus casseliflavus* CIP 103018^{T} mesurant 727 paires de bases :
SEQ.ID. n°28 : séquence partielle du gène *rpoB* chez *Enterococcus saccharolyticus* CIP 103246^{T} mesurant 721 paires de bases :
SEQ.ID. n°29 : séquence partielle du gène *rpoB* chez *Enterococcus faecium* CIP 103014^{T} mesurant 727 paires de bases :
SEQ.ID. n°30 : séquence partielle du gène *rpoB* chez *Enterococcus faecalis* CIP 103015^{T} mesurant 724 paires de bases :
SEQ.ID. n°31 : séquence partielle du gène *rpoB* chez *Enterococcus avium* CIP 103019^{T} mesurant 570 paires de bases :
SEQ.ID. n°32 : séquence partielle du gène *rpoB* chez *Abiotrophia defectiva* CIP 103242^{T} mesurant 732 paires de bases :
SEQ.ID. n°33 : séquence partielle du gène *rpoB* chez *Gemella morbilorum* CIP 81.10^{T} mesurant 727 paires de bases :
SEQ.ID. n°34 : séquence partielle du gène *rpoB* chez *Gemella haemolysans* CIP 101126^{T} mesurant 726 paires de bases :
SEQ.ID. n°35 : séquence partielle du gène *rpoB* chez *Granulicatella adjacens* CIP 103243^{T} mesurant 719 paires de bases :

Dans les séquences ci-dessus, le nucléotide M désigne A ou C, le nucléotide R désigne A ou G, le nucléotide W désigne A ou T, le nucléotide Y désigne C ou T et le nucléotide N désigne A, T, C ou G.

Dans les séquences ci-dessus, les références CIP se rapportent à des dépôts à la Collection Nationale de Culture des Microorganismes (CNCM) de l'Institut Pasteur à Paris (France).

### Exemple 3. Identification en aveugle d'une collection de 20 souches bactériennes comprenant 10 souches de bactéries appartenant aux genres Streptococcus et genres apparentés.

Une collection de vingt souches appartenant aux espèces bactériennes suivantes: *Steptococcus pyogenes, Streptococcus sanguis, Granulicatella adjacens, Abiotrophia defectiva, Enterococcus avium, Enterococcus faecalis, Gemella haemolysans, Gemella morbilorum, Streptococcus equi, Streptococcus anginosus, Staphylococcus aureus, Pseudomohas oleovorans, Mycobacterium avium, Bacillus cereus, Acinetobacter anitratus, Corynebacterium amycolatum, Klebsiella terrigena, Pasteurella, Lactobacillus rhamnosus, Staphylococcus*, a été codée de façon à réaliser une identification moléculaire en aveugle (l'expérimentateur ne connaissant pas a priori l'identité des souches) des souches selon le procédé décrit dans la présente demande de brevet. L'extraction des acides nucléiques ainsi que l'amplification du fragment du gène *rpoB* ont été réalisées comme décrites dans l'exemple n°2 en incorporant des amorces consistant dans des mélanges de 4 oligonucléotides qui ont des séquences consistant dans les séquences SEQ ID N°6 (comme amorce 5')° et SEQ ID N°7 (comme amorce 3') avec N représentant l'inosine, dans une amplification PCR (Fig. 1). Le séquençage de ces 10 amplifiats a été réalisé en incorporant dans la réaction de séquençage les amorces SEQ ID N°6 et SEQ ID N°7 comme décrit dans l'exemple n°2 et la comparaison des séquences obtenues avec les séquences SEQ.ID n° 1 à 5 et 8 à 35 a permis d'identifier les dix souches amplifiées comme étant *Steptococcus pyogenes, Streptococcus sanguis, Granulicatella adjacens, Abiotrophia defectiva, Enterococcus avium, Enterococcus faecalis, Gemella haemolysans, Gemella morbilorum, Streptococcus equi, Streptococcus anginosus*. Le décodage de ces 10 souches a montré 100% de concordance entre l'identification moléculaire selon le procédé faisant l'objet de la présente invention et l'identification établie antérieurement par les méthodes phénotypiques standard. Ce résultat illustre la spécificité du jeu d'amorces SEQ ID N°6/SEQ ID N° 7.

Les autres bactéries choisies pour ce qu'elles sont fréquemment isolées dans les prélèvements cliniques humains ou animaux susceptibles de contenir également des bactéries du genre *Streptococcus,* n'ont pas été amplifiées, démontrant ainsi la spécificité des amorces utilisées pour le genre *Streptococcus* et dits 4 genres apparentés dans les conditions d'utilisation pour la détection des bactéries du genre *Streptococcus* et dits 4 genres apparentés selon l'invention par rapport aux bactéries d'un autre genre.

Sur la figure 1 sont représentés les produits d'amplification PCR obtenus à partir de dix souches bactériennes codées, comportant 7 souches appartenant au genre *Streptococcus* et lesdits 4 genres apparentés (colonnes 2, 3, 4, 7 -11) et 3 souches bactériennes de genres bactériens autres que *Streptococcus* et lesdits 4 genres apparentés (colonnes 5,6 et 12). Les colonnes 1 et 13 représentent le marqueur de poids moléculaire. Les produits d'amplification sont obtenus après incorporation des amorces SEQ ID N°6 et SEQ ID N° 7 décrits ci-dessus et sont visualisés par coloration au bromure d'éthidium après électrophorèse sur un gel d'agarose.

## Revendications

1. Fragment d'un gène rpoB d'une bactérie du genre Streptococcus et des 4 genres apparentés enterococcus, Gemella, Abiotrophia et Granulicatella, **caractérisé en ce que** sa séquence consiste dans l'une des séquences SEQ.ID. n° 8 à 35, et les séquences complémentaires.

2. Oligonucléotide **caractérisé en ce qu'**il consiste en une séquence spécifique d'une espèce d'une bactérie du genre Streptococcus et des 4 genres apparentés enterococcus, Gemella, Abiotrophia et Granulicatella, de 20 à 100 nucléotides consécutifs, de préférence au moins 30 nucléotides consécutifs, inclus dans l'une des dites séquences SEQ ID n°8 à 35 et les séquences complémentaires.

3. Utilisation d'un fragment de gène ou oligonucléotide selon l'une des revendications 1 ou 2, à titre de sonde d'espèce d'une bactérie du genre *Streptococcus* et dits genres apparentés.

4. Oligonucléotide **caractérisé en ce qu'**il comprend une séquence d'au moins 8, de préférence au moins 12, de préférence encore 18 à 35 motifs nucléotidiques, dont au moins une séquence de 8 motifs nucléotidiques consécutifs inclus dans l'une des séquences SEQ.ID. n° 6 et 7 suivantes :
- SEQ ID N° 6 : 5'-AARYTNGGMCCTGAAGAAAT-3', et
- SEQ ID N°7: 5'-TGNARTTTRTCATCAACCATGTG-3',
dans lesquelles :
- N représente l'inosine ou l'un des 4 nucléotides A, T, C ou G,
- R représente A ou G,
- M représente A ou C, et
- Y représente C ou T,
et les séquences complémentaires.

5. Mélange d'oligonucléotides, **caractérisé en ce qu'**il est constitué d'un mélange équimolaire d'oligonucléotides selon la revendication 4, ayant tous une séquence différente et comprenant tous une séquence incluse dans SEQ ID n°6 ou tous une séquence incluse dans SEQ ID n°7.

6. Mélange d'oligonucléotides, **caractérisé en ce qu'**il est constitué d'un mélange équimolaire de 32 oligonucléotides selon la revendication 4, de séquences différentes comprenant chacune au moins 15, de préférence au moins 18 motifs nucléotidiques consécutifs, inclus dans la séquence suivante :
- SEQ ID N° 6 : 5'-AARYTNGGMCCTGAAGAAAT-3',
dans laquelle :
- R représente A ou G,
- Y représente C ou T,
- M représente A ou C, et
- N représente A, T, C ou G, et les séquences complémentaires.

7. Mélange d'oligonucléotides, **caractérisé en ce qu'**il est constitué d'un mélange équimolaire de 8 oligonucléotides selon la revendication 4 de séquences différentes comprenant chacune au moins 15, de préférence au moins 18 motifs nucléotidiques consécutifs inclus dans la séquence suivante :
- SEQ ID N° 6 : 5'-AARYTNGGMCCTGAAGAAAT-3',
dans laquelle :
- R représente A ou G,
- Y représente C ou T,
- M représente A ou C, et
- N représente l'inosine, et les séquences complémentaires.

8. Mélange d'oligonucléotides, **caractérisé en ce qu'**il est constitué d'un mélange équimolaire de 16 oligonucléotides selon la revendication 4, de séquences différentes comprenant chacune au moins 15, de préférence au moins 21 motifs nucléotidiques consécutifs inclus dans la séquence suivante :
- SEQ ID N°7: 5'-TGNARTTTRTCATCAACCATGTG-3',
dans laquelle :
- R représente A ou G, et
- N représente A, T, C ou G, et les séquences complémentaires.

9. Mélange d'oligonucléotides, **caractérisé en ce qu'**il est constitué d'un mélange équimolaire de 4 oligonucléotides selon la revendication 4, de séquences différentes comprenant chacune au moins 15, de préférence au moins 21 motifs nucléotidiques consécutifs inclus dans la séquence suivante :
- SEQ ID N°7: 5'-TGNARTTTRTCATCAACCATGTG-3',
dans laquelle :
- R représente A ou G, et
- N représente l'inosine,
et les séquences complémentaires.

10. Mélange d'oligonucléotides selon la revendication 5, **caractérisé en ce que** lesdites séquences consistent dans les séquences SEQ.ID. n° 6 et 7 dans lesquelles, de préférence, N représente l'inosine, et les séquences complémentaires.

11. Utilisation d'un oligonucléotide ou mélange d'oligonucléotides selon l'une des revendications 4 à 10, à titre d'amorce d'amplification ou sonde de genre d'une bactérie du genre *Streptococcus* et des 4 genres apparentés ***Enterococcus, Gemella, Abiotrophia et Granulicatella.***

12. Procédé de détection par identification moléculaire d'une bactérie de l'une des espèces du genre *Streptococcus* et des 4 genres apparentés *Enterococcus, Gemella, Abiotrophia et Granulicatella*, **caractérisé en ce qu'**on utilise :
- un fragment de gène *rpoB* ou un oligonucléotide selon l'une des revendications 1 ou 2, et/ou
- au moins un oligonucléotide ou mélange d'oligonucléotides selon l'une des revendications 4 à 10.

13. Procédé selon la revendication 12 dans lequel on cherche à détecter la présence d'une bactérie du genre *Streptococcus* ou desdits 4 genres apparentés, **caractérisé en ce qu'**il comprend les étapes dans lesquelles:
1- on met en contact au moins une sonde de genre comprenant un dit mélange d'oligonucléotides selon l'une des revendications 5 à 10, avec un échantillon contenant ou susceptible de contenir des acides nucléiques d'au moins une telle bactérie du genre *Streptococcus* et ses dits 4 genres apparentés, et
2- on détermine la formation ou l'absence de formation d'un complexe d'hybridation entre ladite sonde de genre et les acides nucléiques de l'échantillon, et on détermine ainsi la présence de ladite bactérie dans l'échantillon s'il y a formation d'un complexe d'hybridation.

14. Procédé selon la revendication 12, **caractérisé en ce qu'**il comprend les étapes dans lesquelles :
1- on met en contact les amorces d'amplification comprenant desdits mélanges d'oligonucléotides selon l'une des revendications 5 à 10, avec un échantillon contenant ou susceptible de contenir des acides nucléiques d'au moins une telle bactérie du genre *Streptococcus* et desdits 4 genres apparentés, et avec :
- comme amorce 5', un dit mélange d'oligonucléotides comprenant une séquence incluse dans la séquence SEQ.ID. n°6, de préférence consistant dans ladite séquence SEQ ID N°6 complète, ou une dite séquence complémentaire selon l'une des revendications 5, 6, 7 ou 10, et
- comme amorce 3' un dit mélange d'oligonucléotides comprenant une séquence incluse dans la séquence SEQ.ID. n°7, ou de préférence consistant dans ladite séquence SEQ ID N°7 complète ou une séquence complémentaire selon l'une des revendications 5, 8, 9 ou 10,
2- on réalise une amplification d'acides nucléiques par réaction de polymérisation enzymatique et on détermine l'apparition ou l'absence d'un produit d'amplification, et on détermine ainsi la présence de ladite bactérie dans l'échantillon si un produit d'amplification est apparu.

15. Procédé selon la revendication 12 ou 14, **caractérisé en ce qu'**on cherche à détecter spécifiquement une espèce donnée d'une bactérie du genre *Streptococcus* et desdits 4 genres apparentés choisie parmi les espèces :
*Streptococcus mutans, Streptococcus oralis, Streptococcus pneumoniae, Streptococcus pyogenes, Streptococcus salivarius, Streptococcus sanguinis, Streptococcus suis, Streptococcus acidominimus, Streptococcus agalactiae, Streptococcus anginosus, Streptococcus constellatus, Streptococcus difficilis, Streptococcus dysgalactiae, Streptococcus equi, Streptococcus equinus, Streptococcus intermedius, Streptococcus mitis, Streptococcus bovis, Streptococcus alactolyticus, Streptococcus gallolyticus, Streptococcus macedonicus, Streptococcus infantarius, Streptococcus hominis, Granulicatella adjacens, Abiotrophia defectiva, Enterococcus avium, Enterococcus casselliflavus, Enterococcus faecalis, Enterococcus faecium, Enterococcus gallinarum, Enterococcus sacharolyticus, Gemella haemolysans et Gemella morbillorum*,
procédé dans lequel :
1- on met en contact un échantillon contenant ou susceptible de contenir des acides nucléiques d'au moins une telle bactérie, avec au moins une sonde d'espèce consistant dans un fragment de gène ou un oligonucléotide selon l'une des revendications 1 ou 2, et
2- on détermine la formation ou l'absence d'un complexe d'hybridation entre ladite sonde et les acides nucléiques de l'échantillon, et on détermine ainsi la présence de ladite bactérie dans l'échantillon s'il y a formation d'un complexe d'hybridation.

16. Procédé selon la revendication 12, **caractérisé en ce qu'**on cherche à détecter une espèce donnée d'une bactérie du genre *Streptococcus* et desdits 4 genres apparentés choisie parmi les espèces : *Streptococcus mutans, Streptococcus oralis, Streptococcus pneumoniae, Streptococcus pyogenes, Streptococcus salivarius, Streptococcus sanguinis, Streptococcus suis, Streptococcus acidominimus, Streptococcus agalactiae, Streptococcus anginosus, Streptococcus constellatus, Streptococcus difficilis, Streptococcus dysgalactiae, Streptococcus equi, Streptococcus equinus, Streptococcus intermedius, Streptococcus mitis, Streptococcus bovis, Streptococcus alactolyticus, Streptococcus gallolyticus, Streptococcus macedonicus, Streptococcus infantarius, Streptococcus hominis, Granulicatella adjacens, Abiotrophia defectiva, Enterococcus avium, Enterococcus casselliflavus, Enterococcus faecalis, Enterococcus faecium, Enterococcus gallinarum, Enterococcus sacharolyticus, Gemella haemolysans et Gemella morbillorum,* procédé dans lequel, dans un échantillon contenant ou susceptible de contenir des acides nucléiques d'au moins une telle bactérie du genre *Streptococcus et desdits 4 genres apparentés,* on effectue les étapes dans lesquelles :
a) on réalise une réaction de séquençage d'un fragment du gène *rpoB* amplifié d'une dite bactérie donnée à l'aide des amorces nucléotidiques consistant dans desdits mélanges d'oligonucléotides selon l'une des revendications 5 à 10 comprenant des séquences incluses dans la séquence SEQ.ID. n° 6 comme amorce 5' et SEQ.ID.n° 7 comme amorce 3', de préférence des séquences consistant dans lesdites séquences SEQ.ID. n° 6 et 7, et lesdites séquences complémentaires, et
b) on détermine la présence ou l'absence de l'espèce donnée de ladite bactérie en comparant la séquence dudit fragment obtenu avec la séquence d'un fragment du gène *rpoB* de ladite bactérie comprenant respectivement lesdites séquences SEQ ID n° 8 à 35 selon l'une des revendications 1 ou 2 et séquences complémentaires, et on détermine ainsi la présence de ladite bactérie dans l'échantillon si la séquence du fragment obtenue est identique à la séquence connue du gène ou du fragment de gène *rpoB* de ladite bactérie.

17. Trousse de diagnostic utile dans un procédé selon l'une des revendications 12 à 16, **caractérisée en ce qu'**elle comprend au moins un dit oligonucléotide, mélange d'oligonucléotides, ou fragment de gène selon l'une des revendications 1 ou 2 et 4 à 10.

## Claims

1. *rpoB* gene fragment of a bacterium of the genus *Streptococcus* and the 4 related genera *Enterococcus, Gemella, Abiotrophia* and *Granulicatella,* **characterized in that** its sequence consists in a sequence chosen from among sequences SEQ ID n° 8 to 35 and their complementary sequences.

2. Oligonucleotide **characterized in that** it consists in a sequence specific to a species of a bacterium of genus *Streptococcus* and the 4 related genera *Enterococcus, Gemella, Abiotrophia* and *Granulicatella,* having from 20 to 100 consecutive nucleotides, preferably at least 30 consecutive nucleotides included in one of said sequences SEQ ID n° 8 to 35 and the complementary sequences.

3. Use of a gene, fragment or oligonucleotide as defined in any of claims 1 or 2 as species probe for a bacterium of genus *Streptococcus* and said related genera.

4. Oligonucleotide **characterized in that** it comprises a sequence of at least 8, preferably at least 12, further preferably 18 to 35 nucleotide motifs, including at least one sequence of 8 consecutive nucleotide motifs included in one of the following sequences SEQ ID n° 6 and 7:
- SEQ ID n° 6: 5'- AARYTNGGMCCTGAAGAAAT-3', and
- SEQ ID n° 7: 5'- TGNARTTTRTCATCAACCATGTG-3'
in which:
- N represents inosine or one of the 4 nucleotides A, T, C or G,
- R represents A or G,
- M represents A or C, and
- Y represents C or T,
and the complementary sequences.

5. Mixture of oligonucleotides, **characterized in that** it consists of an equimolar mixture of oligonucleotides as defined in claim 4, all having a different sequence and all comprising a sequence included in SEQ ID n° 6 or all a sequence included in SEQ ID n° 7.

6. Mixture of oligonucleotides, **characterized in that** it consists of an equimolar mixture of 32 oligonucleotides as defined in claim 4, having different sequences and each comprising at least 15, preferably at least 18 consecutive nucleotide motifs, included in the following sequence:
- SEQ ID n°6: 5'- AARYTNGGMCCTGAAGAAAT-3'
in which:
- R represents A or G,
- Y represents C or T,
- M represents A or C, and
- N represents A, T, C or G
and the complementary sequences.

7. Mixture of oligonucleotides, **characterized in that** it consists of an equimolar mixture of 8 oligonucleotides as defined in claim 4, having different sequences and each comprising at least 15, preferably at least 18 consecutive nucleotide motifs included in the following sequence:
- SEQ ID n°6: 5'- AARYTNGGMCCTGAAGAAAT-3'
in which:
- R represents A or G,
- Y represents C or T,
- M represents A or C, and
- N represents inosine, and the complementary sequences.

8. Mixture of oligonucleotides **characterized in that** it consists of an equimolar mixture of 16 oligonucleotides as defined in claim 4, having different sequences and each comprising at least 15, preferably at least 21 consecutive nucleotide motifs included in the following sequence:
- SEQ ID n°7: 5'- TGNARTTTRTCATCAACCATGTG-3'
in which:
- R represents A or G, and
- N represents A, T, C or G and the complementary sequences.

9. Mixture of oligonucleotides, **characterized in that** it consists of an equimolar mixture of 4 oligonucleotides as defined in claim 4, having different sequences and each comprising at least 15, preferably at least 21 consecutive nucleotide motifs included in the following sequence:
- SEQ ID n°7: 5'- TGNARTTTRTCATCAACCATGTG-3'
in which:
- R represents A or G, and
- N represents inosine
and the complementary sequences.

10. Mixture of oligonucleotides as defined in claim 5, **characterized in that** said sequences consist of sequences SEQ ID n°6 and 7 in which, preferably, N represents inosine, and the complementary sequences.

11. Use of an oligonucleotide or mixture of oligonucleotides as in any of claims 4 to 10, as amplification primer or genus probe for a bacterium of genus *Streptococcus* and the 4 related genera *Enterococcus, Gemella, Abiotrophia* and *Granulicatella*.

12. Detection method by molecular identification to detect a bacterium of one of the species of genus *Streptococcus* and the 4 related genera *Enterococcus, Gemella, Abiotrophia* and *Granulicatella,* **characterized in that** the following is used:
- an *rpoB* gene fragment or an oligonucleotide as in any of claims 1 or 2, and/or
- at least one oligonucleotide or mixture of oligonucleotides as in any of claims 4 to 10.

13. Method as in claim 12 in which it is sought to detect the presence of a bacterium of genus *Streptococcus* or of said 4 related genera, **characterized in that** it comprises the steps in which:
1- at least one genus probe comprising a said mixture of oligonucleotides as in any of claims 7 to 12, is contacted with a specimen containing or possibly containing nucleic acids of at least one such bacterium of genus *Streptococcus* and its said 4 related genera, and
2- the formation or non-formation of a hybridisation complex is determined between said genus probe and the nucleic acids of the specimen, and in this way the presence is determined of said bacterium in the specimen if a hybridisation complex is formed.

14. Method as in claim 12, **characterized in that** it comprises the steps in which:
1- the amplification primers comprising said mixtures of oligonucleotides as in any of claims 5 to 10 are contacted with a specimen containing or possibly containing nucleic acids of at least one such bacterium of genus *Streptococcus* and said 4 related genera, and with:
- as 5' primer, a said mixture of oligonucleotides comprising a sequence included in sequence SEQ ID n°6, preferably consisting of said complete sequence SEQ ID n°6, or a said complementary sequence as in any of claims 5, 6, 7 or 10, and
- as 3' primer a said mixture of oligonucleotides comprising a sequence included in sequence SEQ ID n°7, or preferably consisting of said complete sequence SEQ ID n°7, or a complementary sequence as in any of claims 5, 8, 9 or 10.
2- the nucleic acids are amplified by enzymatic polymerisation reaction to determine the presence or absence of an amplification product, and in this manner the presence is determined of said bacterium in the specimen if an amplification product 15.

15. Method as in claim 12 or 13, **characterized in that** it is sought to specifically detect a given species of a bacterium in the *Streptococcus* genus and said 4 related genera, chosen from among the species:
*Streptococcus mutans, Streptococcus oralis, Streptococcus pneumoniae, Streptococcus pyogenes, Streptococcus salivarius, Streptococcus sanguinis, Streptococcus suis, Streptococcus acidominimus, Streptococcus agalactiae, Streptococcus anginosus, Streptococcus constellatus, Streptococcus difficilis, Streptococcus dysgalactiae, Streptococcus equi, Streptococcus equinus, Streptococcus intermedius, Streptococcus mitis, Streptococcus bovis, Streptococcus alactolyticus, Streptococcus gallolyticus, Streptococcus macedonicus, Streptococcus infantarius, Streptococcus hominis, Granulicatella adjacens, Abiotrophia defectiva, Enterococcus avium, Enterococcus casselliflavus, Enterococcus faecalis, Enterococcus faecium, Enterococcus gallinarum, Enterococcus sacharolyticus, Gemella haemolysans*, and *Gemella morbillorum*,
method in which:
1- a specimen containing or possibly containing nucleic acids of at least one such bacterium is contacted with at least one species probe consisting of a gene fragment or an oligonucleotide as in claim 1 or 2, and
2- the formation or non-formation is determined of a hybridisation complex between said probe and the nucleic acids of the specimen, and in this way the presence of said bacterium in the sample is determined if a hybridisation complex is formed.

16. Method as in claim 12, **characterized in that** it is sought to detect a given species of a bacterium of genus *Streptococcus* and said related genera chosen from among the species:
*Streptococcus mutans, Streptococcus oralis, Streptococcus pneumoniae, Streptococcus pyogenes, Streptococcus salivarius, Streptococcus sanguinis, Streptococcus suis, Streptococcus acidominimus, Streptococcus agalactiae, Streptococcus anginosus, Streptococcus constellatus, Streptococcus difficilis, Streptococcus dysgalactiae, Streptococcus equi, Streptococcus equinus, Streptococcus intermedius, Streptococcus mitis, Streptococcus bovis, Streptococcus alactolyticus, Streptococcus gallolyticus, Streptococcus macedonicus, Streptococcus infantarius, Streptococcus hominis, Granulicatella adjacens, Abiotrophia defectiva, Enterococcus avium, Enterococcus casselliflavus, Enterococcus faecalis, Enterococcus faecium, Enterococcus gallinarum, Enterococcus sacharolyticus, Gemella haemolysans*, and *Gemella morbillorum*, a method in which, in a specimen containing or possibly containing nucleic acids of at least one said bacterium of said genus *Streptococcus* and said 4 related genera, the steps are performed in which:
a) a sequencing reaction is conducted of an amplified *rpoB* gene fragment of a said given bacterium using nucleotide primers consisting of said oligonucleotide mixtures as in any of claims 5 to 10 comprising sequences included in sequence SEQ ID n°6 as 5' primer and in SEQ ID n°7 as 3' primer, preferably sequences consisting of said sequences SEQ ID n° 6 and 7, and said complementary sequences, and
b) the presence or absence of the given species of said bacterium is determined by comparing the sequence of said obtained fragment with the sequence of the complete *rpoB* gene of said bacterium or the sequence of a *rpoB* gene fragment of said bacterium respectively comprising said sequences SEQ ID n°8 to 35 as in any of claims 1 or 2 and complementary sequences, and in this manner the presence of said bacterium in the specimen is determined if the obtained sequence of said fragment is identical to the known sequence of the rpoB gene or gene fragment of said bacterium.

17. Diagnosis kit for use in a method as in any of claims 12 to 16, **characterized in that** it comprises at least one said oligonucleotide, mixture of oligonucleotides, or gene fragment as in any of claims 1 or 2 and 4 to 10.

## Patentansprüche

1. Fragment eines rpoB-Gens eines Bakteriums der Gattung Streptococcus und der vier verwandten Gattungen Enterococcus, Gemella, Abiotrophia und Granulicatella, **dadurch gekennzeichnet, daß** seine Sequenz aus einer der Sequenzen SEQ ID No 8 bis 35 und den komplementären Sequenzen besteht.

2. Oligonukleotid, **dadurch gekennzeichnet, daß** es aus einer spezifischen Sequenz einer Spezies eines Bakteriums der Gattung Streptococcus und der vier verwandten Gattungen Enterococcus, Gemella, Abiotrophia und Granulicatella, von 20 bis 100 aufeinanderfolgenden Nukleotiden, vorzugsweise wenigstens 30 aufeinanderfolgenden Nukleotiden, enthalten in einer der genannten Sequenzen SEQ ID No 8 bis 35 und den komplementären Sequenzen besteht.

3. Verwendung eines Genfragments oder Oligonukleotids nach einem der Ansprüche 1 oder 2, als Speziessonde eines Bakteriums der Gattung Streptococcus und der genannten verwandten Gattungen.

4. Oligonukleotid, **dadurch gekennzeichnet, daß** es eine Sequenz von wenigstens 8, vorzugsweise wenigstens 12, weiterhin vorzugsweise 18 bis 35 Nukleotidmustern umfaßt, darunter wenigstens eine Sequenz von 8 aufeinanderfolgenden Nukleotidmustern, enthalten in einer der folgenden Sequenzen SEQ ID No 6 und 7:
- SEQ ID No 6: 5'-AARYTNGGMCCTGAAGAAAT-3', und
- SEQ ID No 7: 5'-TGNARTTTRTCATCAACCATGTG-3',
worin:
- N Inosin oder eines der 4 Nukleotide A, T, C oder G darstellt,
- R A oder G darstellt,
- M A oder C darstellt, und
- Y C oder T darstellt,
sowie den komplementären Sequenzen.

5. Oligonukleotidgemisch, **dadurch gekennzeichnet, daß** es aus einer äquimolaren Mischung von Oligonukleotiden nach Anspruch 4 besteht, die alle eine unterschiedliche Sequenz haben und die alle eine Sequenz umfassen, welche in SEQ ID No 6 enthalten ist oder alle eine Sequenz umfassen, die in SEQ ID No 7 enthalten ist.

6. Oligonukleotidgemisch, **dadurch gekennzeichnet, daß** es aus einer äquimolaren Mischung von 32 Oligonukleotiden nach Anspruch 4, mit unterschiedlichen Sequenzen besteht, die jeweils wenigstens 15, vorzugsweise wenigstens 18 aufeinanderfolgende Nukleotidmuster umfassen, die in der folgenden Sequenz:
- SEQ ID No 6: 5'-AARYTNGGMCCTGAAGAAAT-3',
worin:
- R A oder G darstellt,
- Y C oder T darstellt,
- M A oder C darstellt, und
- N A, T, C oder G darstellt,
sowie den komplementären Sequenzen enthalten sind.

7. Oligonukleotidgemisch, **dadurch gekennzeichnet, daß** es aus einer äquimolaren Mischung von 8 Oligonukleotiden nach Anspruch 4, mit unterschiedlichen Sequenzen besteht, die jeweils wenigstens 15, vorzugsweise wenigstens 18 aufeinanderfolgende Nukleotidmuster umfassen, die in der folgenden Sequenz:
- SEQ ID No 6: 5'-AARYTNGGMCCTGAAGAAAT-3',
worin:
- R A oder G darstellt,
- Y C oder T darstellt,
- M A oder C darstellt, und
- N Inosin darstellt,
sowie den komplementären Sequenzen enthalten sind.

8. Oligonukleotidgemisch, **dadurch gekennzeichnet, daß** es aus einer äquimolaren Mischung von 16 Oligonukleotiden nach Anspruch 4, mit unterschiedlichen Sequenzen besteht, die jeweils wenigstens 15, vorzugsweise wenigstens 21 aufeinanderfolgende Nukleotidmuster umfassen, die in der folgenden Sequenz:
- SEQ ID No 7: 5'-TGNARTTTRTCATCAACCATGTG-3',
worin:
- R A oder G darstellt, und
- N A, T, C oder G darstellt,
sowie den komplementären Sequenzen enthalten sind.

9. Oligonukleotidgemisch, **dadurch gekennzeichnet, daß** es aus einer äquimolaren Mischung von 4 Oligonukleotiden nach Anspruch 4, mit unterschiedlichen Sequenzen besteht, die jeweils wenigstens 15, vorzugsweise wenigstens 21 aufeinanderfolgende Nukleotidmuster umfassen, die in der folgenden Sequenz:
- SEQ ID No 7: 5'-TGNARTTTRTCATCAACCATGTG-3',
worin:
- R A oder G darstellt, und
- N Inosin darstellt,
sowie den komplementären Sequenzen enthalten sind.

10. Oligonukleotidgemisch nach Anspruch 5, **dadurch gekennzeichnet, daß** die Sequenzen aus den Sequenzen SEQ ID No 6 und 7, worin vorzugsweise N Inosin darstellt, und den komplementären Sequenzen bestehen.

11. Verwendung eines Oligonukleotids oder Oligonukleotidgemischs nach einem der Ansprüche 4 bis 10, als Amplifikationsprimer oder Gattungssonde eines Bakteriums der Gattung Streptococcus und der vier verwandten Gattungen Enterococcus, Gemella, Abiotrophia und Granulicatella.

12. Verfahren zur Ermittlung eines Bakteriums von einer der Spezies der Gattung Streptococcus und der vier verwandten Gattungen Enterococcus, Gemella, Abiotrophia und Granulicatella, mittels molekularer Identifikation, **dadurch gekennzeichnet, daß** folgendes verwendet wird:
- ein Fragment eines rpoB-Gens oder ein Oligonukleotid nach einem der Ansprüche 1 oder 2, und/oder
- wenigstens ein Oligonukleotid oder Oligonukleotidgemisch nach einem der Ansprüche 4 bis 10.

13. Verfahren nach Anspruch 12, bei dem versucht wird, das Vorliegen eines Bakteriums der Gattung Streptococcus oder der vier verwandten Gattungen zu ermitteln, **dadurch gekennzeichnet, daß** es die Schritte umfaßt, bei denen:
1- wenigstens eine Gattungssonde, die ein Oligonukleotidgemisch nach einem der Ansprüche 5 bis 10 umfaßt, mit einer Probe in Kontakt gebracht wird, die Nukleinsäuren von wenigstens einem solchen Bakterium der Gattung Streptococcus und ihrer vier verwandten Gattungen enthält oder enthalten kann, und
2- die Bildung oder die Nichtbildung eines Hybridisierungskomplexes zwischen der Gattungssonde und den Nukleinsäuren der Probe ermittelt wird und so das Vorliegen des Bakteriums in der Probe bestimmt wird, wenn es zur Bildung eines Hybridisierungskomplexes kommt.

14. Verfahren nach Anspruch 12, **dadurch gekennzeichnet, daß** es die Schritte umfaßt, bei denen:
1- die Amplifikationsprimer, welche Oligonukleotidgemische nach einem der Ansprüche 5 bis 10 umfassen, mit einer Probe in Kontakt gebracht werden, die Nukleinsäuren von wenigstens einem solchen Bakterium der Gattung Streptococcus und der vier verwandten Gattungen enthält oder enthalten kann, sowie mit:
- als 5'-Primer, einem Oligonukleotidgemisch, umfassend eine Sequenz, die in der Sequenz SEQ ID No 6 enthalten ist, vorzugsweise aus der vollständigen Sequenz SEQ ID No 6 besteht, oder eine komplementäre Sequenz, nach einem der Ansprüche 5, 6, 7 oder 10, und
- als 3'-Primer, einem Oligonukleotidgemisch, umfassend eine Sequenz, die in der Sequenz SEQ ID No 7 enthalten ist, oder vorzugsweise aus der vollständigen Sequenz SEQ ID No 7 besteht, oder eine komplementäre Sequenz, nach einem der Ansprüche 5, 8, 9 oder 10,
2- eine Amplifikation von Nukleinsäuren durch enzymatische Polymerisationsreaktion durchgeführt wird und das Auftreten oder Nichtvorliegen eines Amplifikationsproduktes und somit das Vorliegen des Bakteriums in der Probe ermittelt wird, wenn ein Amplifikationsprodukt aufgetreten ist.

15. Verfahren nach Anspruch 12 oder 14, **dadurch gekennzeichnet, daß** versucht wird, eine gegebene Spezies eines Bakteriums der Gattung Streptococcus und der vier verwandten Gattungen spezifisch zu ermitteln, welche aus den folgenden Spezies ausgewählt ist:
Streptococcus mutans, Streptococcus oralis, Streptococcus pneumoniae, Streptococcus pyogenes, Streptococcus salivarius, Streptococcus sanguinis, Streptococcus suis, Streptococcus acidominimus, Streptococcus agalactiae, Streptococcus anginosus, Streptococcus constellatus, Streptococcus difficilis, Streptococcus dysgalactiae, Streptococcus qui, Streptococcus equinus, Streptococcus intermedius, Streptococcus mitis, Streptococcus bovis, Streptococcus alactolyticus, Streptococcus gallolyticus, Streptococcus macedonicus, Streptococcus infantarius, Streptococcus hominis, Granulicatella adjacens, Abiotrophia defectiva, Enterococcus avium, Enterococcus casselliflavus, Enterococcus faecalis, Enterococcus faecium, Enterococcus gallinarum, Enterococcus sacharolyticus, Gemella haemolysans und Gemella morbillorum,
Verfahren, bei dem:
1- eine Probe, die Nukleinsäuren wenigstens eines solchen Bakteriums enthält oder enthalten kann, mit wenigstens einer Speziessonde, die aus einem Genfragment oder einem Oligonukleotid nach einem der Ansprüche 1 oder 2 besteht, in Kontakt gebracht wird, und
2- die Bildung oder das Nichtvorliegen eines Hybridisierungskomplexes zwischen der Sonde und den Nukleinsäuren der Probe ermittelt und somit das Vorliegen des Bakteriums in der Probe bestimmt wird, wenn es zur Bildung eines Hybridisierungskomplexes kommt.

16. Verfahren nach Anspruch 12, **dadurch gekennzeichnet, daß** versucht wird, eine gegebene Spezies eines Bakteriums der Gattung Streptococcus und der vier verwandten Gattungen zu ermitteln, welche aus den folgenden Spezies ausgewählt ist:
Streptococcus mutans, Streptococcus oralis, Streptococcus pneumoniae, Streptococcus pyogenes, Streptococcus salivarius, Streptococcus sanguinis, Streptococcus suis, Streptococcus acidominimus, Streptococcus agalactiae, Streptococcus anginosus, Streptococcus constellatus, Streptococcus difficilis, Streptococcus dysgalactiae, Streptococcus qui, Streptococcus equinus, Streptococcus intermedius, Streptococcus mitis, Streptococcus bovis, Streptococcus alactolyticus, Streptococcus gallolyticus, Streptococcus macedonicus, Streptococcus infantarius, Streptococcus hominis, Granulicatella adjacens, Abiotrophia defectiva, Enterococcus avium, Enterococcus casselliflavus, Enterococcus faecalis, Enterococcus faecium, Enterococcus gallinarum, Enterococcus sacharolyticus, Gemella haemolysans und Gemella morbillorum, Verfahren, bei dem in einer Probe, die Nukleinsäuren wenigstens eines solchen Bakteriums der Gattung Streptococcus und der gennanten verwandten Gattungen enthält oder enthalten kann, die folgenden Schritte durchgeführt werden, bei denen:
a) eine Sequenzierungsreaktion eines Fragments des amplifizierten rpoB-Gens eines gegebenen Bakteriums mit Hilfe der Nukleotidprimer durchgeführt wird, die aus den Oligonukleotidgemischen nach einem der Ansprüche 5 bis 10 bestehen, umfassend Sequenzen, die in der Sequenz SEQ ID No 6 als 5'-Primer und SEQ ID No 7 als 3'-Primer enthalten sind, vorzugsweise Sequenzen, die aus den Sequenzen SEQ ID No 6 und 7 bestehen, sowie die komplementären Sequenzen, und
b) das Vorliegen oder das Nichtvorliegen der gegebenen Spezies des Bakteriums **dadurch** ermittelt wird, daß die Sequenz des erhaltenen Fragments mit der Sequenz eines Fragments des rpoB-Gens des Bakteriums, jeweils umfassend die Sequenzen SEQ ID No 8 bis 35 nach einem der Ansprüche 1 oder 2 und die komplementären Sequenzen, verglichen wird, und somit das Vorliegen des Bakteriums in der Probe ermittelt wird, wenn die Sequenz des erhaltenen Fragments mit der bekannten Sequenz des Gens oder des Fragments des rpoB-Gens des Bakteriums identisch ist.

17. Diagnosekit, das bei einem Verfahren nach einem der Ansprüche 12 bis 16 von Nutzen ist, **dadurch gekennzeichnet, daß** es wenigstens ein Oligonukleotid, Oligonukleotidgemisch oder Genfragment nach einem der Ansprüche 1 oder 2 und 4 bis 10 umfaßt.


### SEQUENCE LISTING

<110> UNIVERSITE DE LA MEDITERRANEE (Aix-Marseille II) et CENTRE NATIONA L DE LA RECHERCHE SCIENTIFIQUE - CNRS
   UNIVERSITE DE LA MEDITERRANEE (Aix-Marseille II) et CENTRE NATIONA L DE LA RECHERCHE SCIENTIFIQUE - CNRS
<120> Identification moléculaire des bactéries du genre Strepetococcus
<130> H52 437 cas 10 PCT MD
<160> 52
<170> PatentIn version 3.1
<210> 1
   <211> 4523
   <212> DNA
   <213> Streptococcus anginosus
<220>
   <221> misc_feature
   <222> (266)..(2087)
   <223> n représente a, t, c, g ou i
<220>
   <221> misc_feature
   <222> (266)..(4430)
   <223> n représente a, t, c, g ou i
<220>
   <221> misc_feature
   <222> (4430)..(4503)
   <223> n représente a, t, c, g ou i
<400> 1
   tcatactttt agagtcagat ttagctgctc tttttgtgcc tgttttggga tttttgtcgt     60
   ttgtcatcaa aattaaagat tctgaaaatt actcaaaaag gataaatgaa aattgctact    120
   ctattccatt aatagagaat gtagaaagaa gaaggagtaa aaaacttggc aggacatgaa    180
   gttcaatacg ggaaacaccg tactcgtcgt agtttttcaa gaatcaagga agttcttgat    240
   ttaccaaatt tgattgaaat ccaganggat tcgttcaaag attttcttga ccatggtttg    300
   aaagaagtat ttgaagatgt acttcctatc tcaaacttta cagatacaat ggagctagag    360
   tttgttggtt atgaaattaa aggatctaaa tacactttag aagaagcacg tatccatgat    420
   gccagctatt ctgcacctat ttttgtgact ttccgtttga ttaataaaga aactggtgaa    480
   atcaaaaccc aagaagtgtt ctttggcgat ttcccaatca tgacagaaat gggaactttc    540
   attatcaatg gtggtgagcg gattatcgta tctcagctcg ttcgttctcc aggtgtttac 600
   ttcaacgata aagtaracaa aaatggtaaa gttggttatg gttcaactgy cattcctaac 660
   cgtggagctt ggttagagct ggaaacagac tcaaaagata ttgcttatac tcggattgac 720
   cgtactcgta agattccgtt tacgacactt gttcgtgcgc ttggtttttc tggcgatgat 780
   gaaatctttg acattttcgg cgacagcgat ctcgttcgca acacgattga aaaggatatt 840
   cataaaaatc caatggattc acgtacggat gaagcgctta aagaaatcta tgaacgtctt 900
   cgtccaggtg agcctaaaac agctgatagt tcacgtagtc tattggtcgc tcgtttcttt 960
   gatccacatc gttacgactt ggcggcagtt ggtcgttata aaatcaataa aaaattaaac 1020
   attaaaacac gtttgttaaa tcaaacgatt gcagagcctt tggtagatcc agaaacaggt 1080
   gaaatcttgg ttgaagctgg aacggttatg acgcgtagtg tcattgatag cattgcagaa 1140
   tacttggacg gtgatttgaa taaaatcact tatattccaa atgatgcagc tgtgttaaca 1200
   gagccagttg ttcttcaaaa attcaaagtg gtggcgccaa ctgatccaga tcgtgtggtg 1260
   actattattg gtaatgccaa cccaggagat cgagttcata cgattacgcc agcagatatt 1320
   ttggctgaga tgaattactt cttgaacctc gctgaaggac ttggtcgtgt ggacgatatt 1380
   gaccacttgg gaaatcgtcg gattcgtgcc gttggtgaat tgcttgctaa ccaagtacgt 1440
   cttggcttgt ctcgtatgga gcgaaacgtt cgggagcgca tgagtgtgca agataatgaa 1500
   gtgttgacac cgcaacaaat cattaacatc cgcccagtca cagcagctat caaagaattc 1560
   tttggttcat ctcaattgtc tcaatttatg gaccaacata atccactgtc tgarttgtct 1620
   cacaaacgyc gtttgtccgc cttgggacct ggtggtttga ctcgtgaycg tgctggatat 1680
   gaargtgcgt gacgtgcact acacncacta tggtcgtatg tgtccgattg aaacncctga 1740
   vggaccaaac atcggtttga tcaayaactt gtcttcttat ggtcanttga ataaatatgg 1800
   ctttatccaa acgccgtatc gtaaagtgra tcgtgaaaca ggtctggtca chaatgaaat 1860
   cgtttggttg acagcggang aagaagatga atttattgta gcgcaagcaa attctaaatt 1920
   aacagaagat ggtcgttttg cagaagcgat tgtcatggga cgtcaccaag ggaacaacca 1980
   agaatttcct tcagatcarg trgatttcat ggatgtgtcg cctaagcagg tagttgccgt 2040
   tgcgacagca tgtantccnk ttccytgaaa aygnacgact caarccntgn tstcatgggt 2100
   gccaacatgc aacgtcaagc sgtaccgttg attgatccgc atgcaccata ygywggtana 2160
   tggtatggaa taccaagcag antsaygamt ctggtgcggc tgattantgc mcaacacgac 2220
   ggtaaagttg tmtattytga tgcagccaaa gttgaagttc gtcgtgaaga tggctcactt 2280
   gtatgtntat catagntgac gaaattccgc cgttnaaact gstggtacgt tgmttacaac 2340
   acaacgtagc ggstggtaaa agattggcga tacagntgta aaaaggtgta stttatcgca 2400
   gacggacctt ctatggaaaa aggtgaaatg gcrcttggac aaaayccaat cgttgcttat 2460
   atgacatggg aaggttacaa ctttgaagat gccgttatca tgagtgagcg httagtgaaa 2520
   gacgatgttt acacatctgt tcacttggag gaattcgaat cagaaacacg tgatacwaag 2580
   cttaggmcct gaagaaatca ckcgcgaaat tccaaacgty ggtgaagatg ccnttygasa 2640
   gaccttggac gaaaygggra ttataccgya ttggtgcyga rgttaaagag ggcgacattc 2700
   ttgttggtaa agtcacacca aaaggtgaaa aagatctttc tgctgaagag cgtctcttgc 2760
   acgcaatctt cggtgacaag tcacgtgaag tacgtgatac ytcycttcgt gtaccwcayg 2820
   gtgsygcatg gkgyygtycg tgatgtgaaa atcttwactc gtgcsaacgg tgatgaattg 2880
   caatcwggtg tcaacatgtt ggtacgtgtt wcacntcgct caaaaacgka araycamgtg 2940
   tyggrgataa gatggcyggw cgtcacggaa acaaaggggt tgtttcccgc attgttccag 3000
   ttgaggatat gccgtatctt ccagatggaa caccagttga tattatgttg aacccacttg 3060
   gggtgccatc tcgtatgaat attggtcaag ttatggagct tcacctcggt atggctgctc 3120
   gcaaccttgg cattcacatt gcaacaccag tatttgacgg ggctagctca gatgatcttt 3180
   gggaaaccgt tcgtgaagct ggcatggata gcgatgctaa gacaatcctt tatgatggcc 3240
   gtactggtga gccatttgat aatcgtgtat ccgttggtgt catgtacatg atcaaactcc 3300
   accatatggt tgatgataag ctccatgccc gttccgttgg tccttattca accgttacgc 3360
   aacaacctct tggtggtaaa gcgcagtttg gtggacaacg ttttggagaa atggaagttt 3420
   gggctcttga agcctacggt gcttctaacg tccttcaaga aatcttgact tacaagtcag 3480
   atgacatcaa tggtcgtttg agagcttatg aagccattac caaaggtaag ccaattccaa 3540
   aaccaggtgt tccagaatcc ttccgtgtcc ttgtaaaaga attgcaatca cttggtcttg 3600
   acatgcgtgt ccttgatgaa gacgacaatg aagtcgaact tcgtgacttg gacgaaggca 3660
   tggatgatga tgtgattcat gtagacgatc ttgaaaaagc acgtgaaaaa gcagcacaag 3720
   aagcaaaagc cgcttttgat gctgaaggga aagaataaga actgattcaa tagataataa 3780
   agaaaggtaa gaaatagtgg ttgatgtaaa tcgttttcaa agtatgcaaa tcaccctagc 3840
   ttctcctagt aaagtccgct cttggtctta tggagaagtg aagaaacctg aaacaattaa 3900
   ctaccgcaca ctaaaaccag aacgcgaagg gctttttgat gaagtcatct ttggtcctac 3960
   gaaagactgg gaatgtgcgt gtggaaaata taaacggatt cgttataaag gaatcatttg 4020
   tgaccgttgt ggtgttgaag taactcgtac taaagttcgt cgtgaacgta tgggacatat 4080
   tgagttgaaa gccccagtct cctcatattt ggtattttaa aggaattcca antcgcatgg 4140
   gcntgacctt ggacatgagc cctcgtgctc ttgaagaagt catntanttt gcagcttatg 4200
   tggtgantga ccctaaagat acnccacttg agcacaaatc cattatgaca gagcgggatg 4260
   gttngtgaac gctgacntga atatggccaa ggctcttttg ttgcaaaaat gggtgytgaa 4320
   gcaatccaag atctnntgaa acangtagac ntggaaaaag aaattgcaga gctcaaagat 4380
   gaattaaaaa cggcaagtgg gcaaaagcgc gtaaamgcta anttcgtcgn tnngactctt 4440
   ttcgatnctt tccaaaaatc atggtacaca aaaccagaac tggatggtct taaaccatcn 4500
   ntntcaccgc tcattccaga cac 4523
<210> 2
   <211> 4118
   <212> DNA
   <213> Streptococcus equinus
<400> 2
   cacgcgtggt cgacggcccg ggctggtgaa ttgtcataag ttgtgtagta gtaaattccc    60
   ttatcagtgt tgatgcatga gctataaata gtgtactcat atttgccact ttcatcgaca   120
   tagcaaagtc ctttttgttg ttcaacggat tttaaaatgt ggaagaattg attaacactg   180
   ctttcttctg tttcttcagc cacagaattt aattttgtaa aagtaacttt tacataacgt   240
   gacattgatg ataaatcacc aggcaagcca agtccaccca tgccacggct ataagtttca   300
   agttctaact ctttagcaaa acgattttct gaaacctttg gagatagatg acgatagtta   360
   ttcaaattga ataattgttt atcaaaagtt ggattattag tcaaaacacc tgttgagtta   420
   ttcgtaaact tatagggcac gcgtggtcga cggcccgggc tggtaaagac ttcttggata   480
   acggattaam agaagttttt gaagatgtac ttccgattac aaactttacg gatactatgg   540
   agcttgaatt tgttggttac gaattgaaag agcctaagta tacgcttgaa gaagctcgta   600
   tccacgatgc atcttattca gcacctattt ttgtaacctt ccgtttgatt aataaagaaa   660
   caggagaaat caaaactcaa gaagttttct tcggtgattt cccaattatg actgaaatgg   720
   gtacattcat catcaacggt ggtgaacgta ttatcgtttc tcagttggtt cgttctcctg   780
   gtgtttattt caacgataaa gttgataaaa acggtaaagt tggttacggt tcaactgtaa   840
   tccctaaccg tggagcatgg cttgaattag aaacagattc aaaagatatt gcttacacac   900
   gtatcgaccg tacacgtaaa attccattta caactcttgt acgtgcgctt ggtttctcag   960
   gtgatgatga aatcatggat atctttggtg atagcgaact tgttcgtaac acaatcgaaa 1020
   aagatattca caaaaaccca gcagactcac gtactgacga agctcttaaa gaaatttacg 1080
   aacgccttcg tccaggtgaa ccaaaaacag ctgatagctc acgtagcttg cttgtagctc 1140
   gtttctttga cccacgtcgt tatgacttgg cagctgttgg tcgttacaaa atcaacaaaa 1200
   aacttaacat caagactcgt cttttgaacc aaacaatcgc tgaaaacttg gttgatgctg 1260
   aaactggtga aatccttgtt gaagctggta cagtaatgac acgtgacgtg attgattcaa 1320
   tcgctgatca attggatggt gaccttaaca aatttgttta cacaccaaat gattacgctg 1380
   ttgtcactga acctgttgtt cttcaaaaat tcaaagttgt tgcaccaaac gatccagacc 1440
   gcgttgttac aatcgttggt aacgcaaatc ctgatgacaa agcgcgtgcg cttacaccag 1500
   ctgatatctt ggcagaaatg tcttacttcc ttaaccttgc tgaaggtcta ggtaaagttg 1560
   atgatatcga ccaccttggg aatcgtcgta ttcgtgccgt tggtgaattg cttgctaacc 1620
   aattccgtat tggtcttgct cgtatggaac gtaacgttcg ggaacgtatg tcagttcaag 1680
   acaacgaagt gttgacacca caacaaatca tcaacattcg tcctgttact gcagccgtta 1740
   aagaattctt cggttcatct caattgtcac agttcatgga ccaacacaac ccactttctg 1800
   agttgtctca caaacgtcgt ttgtcagcct taggacctgg tggtttgact cgtgaccgtg 1860
   ctggttatga agttcgtgac gtgcactaca ctcactatgg tcgtatgtgt ccgattgaaa 1920
   ctcctgaagg acctaacatc ggtttgatca ataacttgtc aacatacgga caccttaata 1980
   aatatggttt catccaaaca ccatatcgta aagttgaccg cgctacaggt gtgattacaa 2040
   acgaaatcgt ttggttgact gccgatgaag aagatgaata cacagtagca caggctaact 2100
   caaaacttaa cgaagatgga acatttgctg aagacatcgt tatgggacgt caccaaggta 2160
   ataaccaaga gttcccagca agcgttgttg acttcgtaga cgtttcacct aaacaagtag 2220
   ttgccgttgc gacagcatgt attcctttcc ttgaaaacga tgactctaac cgtgccctta 2280
   tgggtgccaa catgcaacgt caagcggtgc cattgattga tccacacgca ccatatgttg 2340
   gtactggtat ggaatatcaa gcagcccacg actcaggtgc tgcagttatc gctaaacacg 2400
   atggacgcgt tatcttctct gatgctgaaa aagttgaagt tcgtcgcgaa gatggttcac 2460
   ttgatgttta ccacattact aaattccgtc gttctaactc aggtacagct tataaccaac 2520
   atacacttgt taaagttggc gatatcgttg aaaaaggtga cttcatcgct gatggtcctt 2580
   caatggaaaa aggtgaaatg gcccttggtc aaaacccaat cgtcgcttac atgacktggg 2640
   aaggttacaa cttcgaggat gcggttatca tgtctgaacg ccttgtgaaa gatgatgtct 2700
   atacatctgt tcacttggaa gaatacgaat cagaaacacg tgatactaag ttaggccctg 2760
   aagaaatcac tcgcgaaatt ccaaacgttg gtgaagatgc ccttcgcaac ttggacgaaa 2820
   tggggattat ccgtattggt gccgaagtta aagagggcga cattcttgtt ggtaaagtca 2880
   caccaaaagg tgaaaaagat ctttctgctg aagagcgtct cttgcacgca atcttcggtg 2940
   acaagtcacg tgaagtacgt gatacctctc ttcgtgtacc tcacggtgcc gatggtgtcg 3000
   ttcgtgatgt gaaaatcttt actcgtgcca acggtgatga attgcaatca ggtgttaaca 3060
   tgttggttcg tgtttcacat cgctcaaaaa cgtaagatca aggtcggaga taagatggcc 3120
   ggtcgtccac ggtaacaagg gtgtcgtttc acgtaywgta cctgttgagg atatgccata 3180
   tcttccagat ggaacaccag ytgacawcat gttgaaccca ctsggggtgc catcwcgtat 3240
   gaacatcgga caagttatgg agcttcacct tggtatggct gctcgtaacc ttggtattca 3300
   cattgcaaca ccagtctttg atggggcaac ttctgaagac ctttgggata cagttaacga 3360
   agctggtatg gctagcgacg ctaagacagt tctttacgat ggacgtactg gtgaaccatt 3420
   tgataaccgt gtgtcagttg gtgtcatgta catgattaaa cttcaccaca tggttgatga 3480
   taaacttcac gcacgttcag ttggtcctta ctcacttgtt acgcaacaac ctcttggtgg 3540
   taaagcacaa tttggtggac aacgtttcgg tgaaatggaa gtttgggctt tggaagctta 3600
   cggtgcatca aatgttcttc aagaaatctt gacttacaaa tcagatgatg tcaacggtcg 3660
   tcttaaagct tatgaagcca tcactaaagg taaaccaatt ccaaaaccag gtgttccaga 3720
   atcattccga gttcttgtaa aagaattgca atcacttggt cttgacatgc gcgtgcttga 3780
   tgaagatgac aatgaagtag aacttcgtga tcttgatgaa ggtgaagatg acgatgttat 3840
   gcacgttgat gatcttgaaa aagctcgtca aaaacaagaa gcagaagaag cggaaaaagc 3900
   agaagtttct gcagaagaaa acaaataata ggaaagaaca ttcagacatg agagaggcaa 3960
   gacctgcttc tcttggtcag attgtttgat tgagtcctat aacgataaat gatgtcttac 4020
   gaatcatgaa tttgtaagtc atgacagtta gaaagtagcg cagctatttc aaagtcataa 4080
   gaaggtatca tggtgacgta atcgttacag ccggcgtc 4118
<210> 3
   <211> 3425
   <212> DNA
   <213> Abiotrophia defectiva
<400> 3
   atatagggca cgcgtggtcg acggcccggg ctggtcctaa acaacatgta acgtcactcc     60
   gatgagttgg ttctgttgtc ttttttttgc gcttcaaaga ccgaaaaatg tcatttgtca    120
   acaattatta ataattgtaa ccttaatgta aagtggtgtt cttagattat attatagggg 180
   tgaatcgctt gagtcatatc gtgaaatacg gtaaaaaagc tgagcgtcga agctatgcgc 240
   gtatcgacga agtcttagag ttgccgaact tgattgaaat ccaaacggat tcctacaaat 300
   ggttcttgga tgaagggcta aaagtgatgt tcgaggacat ttcgccgatt gtcgaccatt 360
   cggagaactt ggaacttcat tttgtagact atgagttcaa ggaagctaag tatagcttag 420
   aagaagctcg tagccatgac gctaactact caaaaccaat ctatgtaacc ttgcgcctgt 480
   tcaacaaaga gacaggtgaa gtcaaagaac aagaagtctt cttcggggac ttcccaatca 540
   tgaccgaaat ggggaccttc attatcaacg gggcggaacg ggttatcgtt tcccagttgg 600
   tacgttctcc aggtgtctac ttccacgacc gtatggacaa gaaaggccgc cacagctata 660
   cttctacggt tattcctaac cgtggggctt ggttggaatt tgaatcagat gctaagggga 720
   ttgcctacgt ccgcattgac cggacccgga agattccatt gactgtcttg atgcgtgcct 780
   taggttttgg ttcagatgac gagatttatg atatcttcgg ccaatctgag ctcttagact 840
   taactatcga gaaggatgtt cacaaaaaca ttcaagactc tcgtacggaa gaagccttga 900
   aggacattta cgagcgtctc cgtccaggtg aacctaagac cgcagaaagc tcacgtaacc 960
   tcttggttgc gcgcttcttc gacccacgtc gctatgactt agcacctgta ggtcgttata 1020
   agatcaataa aaagctccac ctcaagaacc gtttggttgg cttgactttg gctgaaacct 1080
   tggttaaccc agaaacaggc gaagtgctct ttgaagaagg aacggtcttg gatcaagaac 1140
   gtgttcaagc cctgattcca tacttagagg ctggcttgaa taaggtaacc ctctatcctt 1200
   ctgaagatag tgtggtagct caaccaattg atttacaaat catcaaagtt tattcaccta 1260
   agaacgccga gcaagtgatt aacatcatcg gtaacgggaa cattgagaag attaagtgct 1320
   tgacgccagc tgacattatt gcgtcaatga actactatct ctatttagac caaggaattg 1380
   gtgtgacaga tgatatcgac cacttggcta accgtcgtat tcgttcagtc ggtgaattat 1440
   tgcaaaacca attccgtatc gggctatccc ggatggaacg ggtagtgcgt gaacgtatgt 1500
   cgctccaaga tgttgcgacc atcacaccgc aacaattgat taacattcgt ccagtagtgg 1560
   cggctattaa ggaattcttc ggttcatccc agttgtcaca attcatggac caagttaacc 1620
   cactcgggga attgacccac aaacgtcgtc tgtcagcctt agggcctggt ggtttgacgc 1680
   gggaccgtgc cggctatgaa gtgcgggacg ttcactactc tcactacggc cgtatgtgtc 1740
   caatcgagac gccagaaggt cctaacatcg ggttgattaa cagcttgtct tcttatgcca 1800
   agattaacaa gtatggtttt attgagacgc cttaccgtaa agtggacaaa tcggttacgc 1860
   cacaccgtgt cacgaccgaa attgactacc tagcagcgga cgaggaagac ttgtacgtag 1920
   tagcccaagc caactctaaa ctcaacgaag acgggacctt cgccaatgac ctagttatgg 1980
   cgcgtttccg ttcacaaaac attgaggtta acgttgacca agtagactac atggacgtat 2040
   cgccaaaaca ggttgtcgct gtcgcgactg ctagcattcc gttcttggaa aacgacgact 2100
   ccaaccgggg cttgatgggt gccaacatgc aacgtcaagc tgtgccactt attaatccac 2160
   aatccccact gattgggact gggatggaat ataaggcagc acacgactct ggggctgcgc 2220
   tcttatgtaa gcgcgccggt gaagtggttt atgtcgatgc taacaaggtg cgcgtgcgca 2280
   ctccagaagg tgaagttgac gaataccgtt taaccaagtt tgcacgttct aacgctggga 2340
   cctgttacaa ccaacgtcca atcgtagaat taggcgacca agttgatgcc ttggaaatct 2400
   tagcagatgg tccatctatg caaaatgggg agatggccct cggtcaaaac ccactggtag 2460
   ccttcatgac ttgggaaggg tataactatg aggacgcggt tatcatgtct gaacgtctgg 2520
   tcaaagacga tgtttatacc tctatccaca ttgaagaata tgaatcagag tcccgtgaya 2580
   cyaagttagg ccctgaagaa attacacgcg aaattccaaa cgtgtccgaa gatgccctca 2640
   agtacttaga caaagacggg attatctgta tcggggcgga agtaaaagac ggcgatatct 2700
   tagttggtaa ggtaacacca aaaggtgtga ccgagttgtc tgcggaagaa cgcttgctcc 2760
   atgctatctt cggtgagaag gcgcgtgaag tacgtgatac ttccttgcgt gtgccacacg 2820
   gcgggggcgg gattgtccac gacgttaaaa tctttacccg cgaagctggc gacgaattgg 2880
   caccaggtgt caacaagcta gtccgcgtct acatcgtaca aaaacgtaaa atcaatgaag 2940
   gggataagat ggccggtcgt cacggtaaca aaggggttgt ctcccttatc atgccggaag 3000
   aagatatgcc attcttacca gatggtaccc cagttgatat catgttgaac ccattagggg 3060
   ttccatcccg tatgaacatc gggcaagtcc tagagttaca cttggggatg gctgctcgcg 3120
   aaatgggcat caagattgca acacctgtct ttgacggtgc tagtgaagaa gatgtctggg 3180
   aaacagttaa ggaagccggc ttagaagctg acgctaagac tatcttatat gatggtcgaa 3240
   ccggtgaacc atttgaccgt aaagtctctg ttggggttat gtacatgatt aagttggccc 3300
   acatggtcga tgacaagttg cacgcccgtt caacaggtcc atactctctg gttacccaac 3360
   aaccattggg tggtaaagct caatttggtg ggcaacgttt cggggagatg gaggtttggg 3420
   cccta 3425
<210> 4
   <211> 3198
   <212> DNA
   <213> Streptococcus mutans
<220>
   <221> misc_feature
   <222> (619)..(3193)
   <223> n représente a, t, c, g ou i
<400> 4
   ggaccctttt atgacttctt ggatacaggt ctgaaggaag tttttgaaga tgtgcttcca   60
   atttccaatt tcacagacac tatggaatta gagtttgtgg gttatgagtt gaaagagcct  120
   aagtatacat tggaagaagc acgtgctcat gatgcacatt attctgcccc catctttgtt  180
   actttccgtc tcatcaataa agaaactggt gaaattaaga cacaagaagt attttttggt  240
   gattttccct tgatgactga aatgggtact tttattatta atggtgctga acgtattatc  300
   gtttctcagt tggtacgttc accaggtgtt tattttaatg ataaagtgga taaaaatggg  360
   aaaattggct atggttcaac tgttatccct aaccgcggtg cttggcttga gcttgaaacg  420
   gactctaagg atattgctta tactcgtatt gatcgtactc gtaaaattcc ttttacgacg  480
   ctggttcgtg cactcggttt ttccggggat gatgagatta ttgatatttt tggtgatagc  540
   gaattggttc gtaataccat tgaaaaagat atccataaaa atcctaatga ctctcgtaca  600
   gatgaagctc tcaaggaant tatgaacgtc ttcgtccggg tgaacctaaa acggcagatt  660
   cntcacgcag tcttctgatt gcacgtttct ttgatgcgcg ccgttatgat tagcagctgt  720
   tggccgctat agataataag aagttaaacg tcaaaacggg tctttgaatc aagtcattgg  780
   ctgaaaanna gtagatctga aacaggcgaa attcttgttg aaagctggga ctgaaatgac  840
   acgcagtgta attgattcga ttgcagatta tcttgatgga gatctcaata aaattgttta  900
   tacgccaaat gaatacgctg ttttgacaga acctgttgtt cttcaaaaat tcaaagttat  960
   ggctccaaat gatccagacc gcacggttac tgttattggt aatgccagtc caagatgaca 1020
   aagtacgtca cttgacacca gccgatacgt attagctgaa atgtcttatt tccttaactt 1080
   ggctgagggt ntaggtaaag ttgatgatat tgaccattta ggcaaccgac gtattcgtgc 1140
   tgttggtgaa ttgcttgcta atcaatttcg tattggtttg gcacgtatgg aacgcaatgt 1200
   tcgtgaacgc atgtccgttc aagataatga agtcttaacg ccacaacaga ttattaacat 1260
   tcgccctgta acagcggcaa ttaaagagtt ttttggttct tctcaattgt cacagttcat 1320
   ggaccaacac aatccactgt ctgaattgtc tcataaacgc cgtttgtcag ctttaggtcc 1380
   tggtggttta acacgcgacc gtgctggtta tgaagtccgt gatgtgcact atacgcatta 1440
   tggtcgtatg tgtccaattg aaacgcctga aggaccaaat attggattga ttaataactt 1500
   gtcttcctat ggtcatctta ataaatatgg atttatccaa acaccatacc gtaaagttga 1560
   ccgtgagaca ggtaaagtaa ccaatgaaat cgaatggctt actgctgatg aagaagatga 1620
   attcactgta gctcaggcta actcaaaact caatgaagat ggaagctttg ctgaagaaat 1680
   cgtcatggga cgtcatcaag ggaataacca agagtttcca gcaagttctg ttgaatatat 1740
   ggatgtttct cctaagcagg tagttgcggt agcgacagca tgtattcctt tccttgaaaa 1800
   tgatgactcc aaccgtgccc ttatgggagc taacatgcag cgccaagctg tgccattgat 1860
   tgatcctaaa gcaccttttg ttggaactgg tatggaatat caagcagccc atgattctgg 1920
   agccgctatt atcgctcaac ataatgggaa agtggtttat tccgatgcag ataagattga 1980
   agttcgccgt gaagatggct cactagatgt ttatcatgtt accaaattcc gtcgttctaa 2040
   ctctggaact gcctacaatc aacgtactct tgttagggta ggcgatagtg ttgagaaggg 2100
   ggactttatt gcagatggtc cttctatgga aaagggtgag atggctcttg gacaaaatcc 2160
   agtggttgct tacatgactt gggagggtta caactttgaa gatgctgtta tcatgagcga 2220
   gcgtcttgtc aaggatgatg tttatacttc tgtccattta gaagaatttg aatctgaaac 2280
   tcgtgataca aagcttggac ctgaagaaat tacgcgtgaa atcccaaatg ttggtgaaga 2340
   tgccctgaaa gaccttgatg aaatgggaat tattcgcatt ggtgctgagg ttaaagaagg 2400
   tgatattcta gttggtaaag tgactcctaa aggagaaaaa gatctttctg cagaagaacg 2460
   cctcttgcat gccatttttg gtgacaaatc acgtgaagtt cgtgatactt ctcttcgtgt 2520
   acctcatggt ggcgacggtg ttgtttgtga tgtgaaaatc tttacacgtg ctaatggaga 2580
   tgaacttcaa tcaggtgtta acatgctggt tcgtgtttat atcgctcaaa aacgtaaaat 2640
   caaggtcgga gataagatgg ccggacgtca tggtaacaag ggtgtcgttt cccgtattgt 2700
   accagtggaa gatatgccat atcttccaga tggaacacct gttgatatca tgcttaatcc 2760
   acttggggtg ccatcacgga tgaacattgg gcaagttatg gaactccatc ttggtatggc 2820
   tgctcgtaat ttgggcattc atattgcaac gcctgtcttt gacggagcaa cttctgatga 2880
   tctttgggaa acagtaaaag aagccggtat ggattctgat gctaaaactg ttctttatga 2940
   tggtcgcaca ggggagccgt ttgataatcg tgtatcagtt ggtgttatgt atatgattaa 3000
   acttcaccac atggttgatg ayaaccattt tgtctatgca magwtcagtt ggcccttakt 3060
   caaygawtam tcagasgart tcctgctwgg tgtaaaggct ncaattgtct ttagaggtta 3120
   aggctggtga aataacggta tgctggtatt gatggcaatg ggcaagtgaa tantcaacac 3180
   cggccgtcta cancgtgc 3198
<210> 5
   <211> 3096
   <212> DNA
   <213> Enterococcus faecalis
<400> 5
   gacccttatc aattggtttt tagatgaggg acttcgtgaa atgtttgaag acattttacc      60
   aattgatgat ttccaaggaa acttatcctt agaatttgtt gactatgaat taaaagaacc     120
   aaagtacaca gtagaagaag cccgcgcaca tgatgccaac tattctgcgc cattacatgt     180
   aacattacgt ttaaccaacc gtgaaacagg tgaaattaaa tcccaagaag tcttcttcgg     240
   cgatttccca ttaatgacag aaatgggtac cttcatcatc aacggggcag aacgtgttat     300
   cgtttcccaa ttagttcgtt ctccaggtgt ttacttccat ggaaaagtgg acaaaaacgg     360
   caaagaaggt tttggctcaa cagtcattcc taaccgtggt gcatggttag aaatggaaac     420
   agatgcgaaa gacatttctt atgttcggat tgaccgcaca cgtaaaattc ctttaactgt     480
   gttagttcgt gctttaggtt tcggttcaga tgataccatc ttcgaaattt tcggcgacag     540
   cgaaagctta cgcaacacaa ttgaaaaaga tttacacaaa aacgcaagtg attctcgtac     600
   agaagaaggc ttgaaagaca tttatgaacg tcttcgccca ggcgaaccaa aaacagcaga     660
   tagctcacgt agcttgttaa cttgcacgtt tctttgatcc aaaacgttat gatttggcaa     720
   acgttggtcg ctacaaagtt aacaaaaaat tagacttaaa aacacgtcta ttaaacttaa     780
   ccttagctga aacgctagtt gatccagaaa ctggtgtaaa tcattgtcga aaaaggcaca     840
   gttttaacac actacatcat ggaaacatta aggcrataca ttgacaaacg gcttaaacag     900
   cgtaacttac tatccaagtg aagatgcggt agtaactgaa ccaatgacga tccaagtgat     960
   tcaagttctt tcaccaaaag atcctgaacg tatcgtaaat gtgattggta acggctatcc    1020
   agacgacagc gtaaaaacag ttcgtccagc agatatcgtt gcttcaatga gctacttctt    1080
   caacttaatg gaagatatcg gtaatgtcga tgacatcgac cacttaggta atcgtcgtat    1140
   ccgttcagta ggcgaattat tacaaaacca attccgtatt ggtttagccc gtatggaacg    1200
   tgtggttcgt gaaagaatgt ctattcaaga cacagaaaca ttgacaccac aacaattaat    1260
   taacatccgt ccagtggtag caagtatcaa agaattcttt ggttcttcac agttatcaca    1320
   gttcatggac caaacaaacc cattaggtga gttaacccat aaacgtcgtc tatcagcctt    1380
   agggcctggt ggtttgactc gtgatcgtgc cggttatgaa gttcgtgacg ttcactactc    1440
   tcactatggt cgtatgtgtc caattgaaac gcctgaggga ccaaatatcg ggttgatcaa    1500
   tagcttatct agttatgcga aagtgaataa atttggtttc atcgaaacgc cttatcgccg   1560
   tgttgatcgt gcgacaggcc gtgttactga tcaagtagat tacttaacag cagacatcga   1620
   agaccattat atcgtagcgc aagcgaactc acttttaaat gaagatggca catttgccaa   1680
   tgatgttgtt atggcgcgtc tacaaagtga aaacttagaa gttgccgtag acaaagttga   1740
   ctacatggac gtttcaccaa aacaagtagt cgcagtcgca acagcatgta ttcctttctt   1800
   agaaaacgat gactccaacc gtgccttgat gggtgccaac atgcagcgtc aagcggtgcc   1860
   gttaattcaa ccacgctctc cgtgggtagg tacaggtatg gaatataaat cagcccatga   1920
   ctcaggtgct gctttactat gtaaacatga cggtgtcgta gaattcgtcg atgcaaaaga   1980
   aattcgcgtt cgtcgcgaca atggcgcatt agacaaatat atggttacaa aattccgtcg   2040
   ttctaactca ggaacaagct acaaccaacg cccaattgtt cacttaggtg aaaagttgaa   2100
   aaggcgatac tttaccggat ggaccttcta tggaagaagc gaaatggctt tatggcaaaa   2160
   cgtcttagtt gccttcatga catgggaagg ttacaactac gaggatgcca ttatcatgag   2220
   ccgtcgttta gttaaagacg atgtctacac ttctgtgcat attgaagaat atgaatcaga   2280
   agcacgtgat acaaaattag gacctgaaga aattacccgt gaaattccaa acgttgggga   2340
   agacgcgttg aaagacttag acgaaatggg gattatccgc attggtgctg aagttcaaga   2400
   tggcgactta ctagttggga aagtcacacc taaaggggtc acagaattat ctgcagaaga   2460
   acgtttatta cacgcaatct tcggggaaaa agcccgcgaa gttcgtgata cgtctctccg   2520
   tgtacctcac ggtggcggcg gtatcgttca tgatgtgaaa atctttactc gtgaagctgg   2580
   cgatgaatta tcaccaggtg tcaacatgtt agttcgtgtc tatatcgttc aaaaacgtaa   2640
   aattcacgaa ggagataaaa tggcgggacg tcacggaaat aaaggggttg tttcccgtat   2700
   tatgccggaa gaagatatgc cattcttacc tgacggaaca cctgttgata tcatgttgaa   2760
   cccattaggg gtaccttctc gtatgaatat cggacaagta cttgaattac acttaggtat   2820
   ggctgctcgc caattaggta ttcacgtcgc aacacctgtt ttcgatgggg caaccgatga   2880
   agacgtttgg gaaactgttc gtgaagctgg tatggctagc gatgctaaaa cagttcttta   2940
   cgatggacgt acaggtgaac catttgataa ccgtatttcc gttggtgtca tgtatatgat   3000
   taaattagcc cacatggttg atgacaaatt gcatgctcgt tcaatcggac cttactctct   3060
   tgttacgcaa caaccgttgg gtgtaaagct caattc                             3096
<210> 6
   <211> 20
   <212> DNA
   <213> amorce
<220>
   <221> misc_feature
   <222> (6)..(6)
   <223> n représente a, t, c ou g ou i
<400> 6
   aarytnggmc ctgaagaaat 20
<210> 7
   <211> 23
   <212> DNA
   <213> amorce
<220>
   <221> misc_feature
   <222> (3)..(3)
   <223> n représente i
<220>
   <221> misc_feature
   <222> (3)..(3)
   <223> n représente a, t, c ou g ou i
<400> 7
   tgnartttrt catcaaccat gtg 23
<210> 8
   <211> 709
   <212> DNA
   <213> Streptococcus suis
<400> 8
   cgcgaaattc caaacgttgg tgaagatgcc cttcgcaact tggacgaaat ggggattatc 60
   cgtattggtg ccgaagttaa agagggcgac attcttgttg gtaaagtcac accaaaaggt 120
   gaaaaagatc tttctgctga agagcgtctc ttgcacgcaa tcttcggtga caagtcacgt 180
   gaagtacgtg atacctctct tcgtgtacct cacggtgccg atggtgtcgt tcgtgatgtg 240
   aaaatcttta ctcgtgccaa cggtgatgaa ttgcaatcag gtgttaacat gttggttcgt 300
   gtttacatcg ctcaaaaacg taagatcaag gtcggagata agatggccgg tcgtcacggt 360
   aacaagggtg tcgtttcacg tattgtacct gttgaggata tgccatatct tccagatgga 420
   acaccagttg acatcatgtt gaacccactc ggggtgccat cacgtatgaa catcggtcag 480
   gttatggaac ttcacttggg tatggcggct cgcaacttgg gcatccatat cgcaacacca 540
   gttttcgatg gtgcaagttc agaagacctc tggtcaactg ttaaagaagc aggtatggac 600
   tcagatgcca agaccattct ttacgatgga cgtacaggtg aaccatttga caaccgtgta 660
   tctgttggtg tcatgtacat gatcaagctt caccacatgg ttgatgaca 709
<210> 9
   <211> 725
   <212> DNA
   <213> Streptococcus sanguinis
<400> 9
   tgtcatcaac catgtggtga gcttaatcat gtacatgaca ccgacagata cacggttgtc    60
   aaacggctca ccggtacgtc catcgtaaag aatagtcttg gcatcgctat ccataccagc   120
   ttcacggaca gtatcccaga ggtcttctga gcttgctcca tcaaagaccg gtgtcgcaat   180
   atggatgccc aagttacgtg ctgccatacc aaggtgaagc tccataacct gaccaatgtt   240
   catacgtgat ggtaccccga gtgggttcag catgatatca actggtgttc cgtctggcaa   300
   ataaggcatg tcttccacag gaacgatacg ggatacaacc cccttgtttc cgtgacgacc   360
   agccatctta tctccgacct tgatcttacg tttttgagcg atgtagacac gaaccaacat   420
   attaacgcca gattgcaact catcaccatt agcacgggta aagatcttca cgtcacgaac   480
   cactccatca gcaccgtgcg gcacacgcag agaggtatca cggacttcac gagacttgtc   540
   tccgaagata gcgtgcaaga ggcgctcttc agcagaaaga tctttttcac ccttaggggt   600
   aactttacct acaaggatat cgccttcctt gacttccgcc ccgatgcgga taatacccat   660
   ttcgtccaaa ttgcgtaggg catcttcccc tacgtttgga atttcgcggg taattcttca   720
   ggtca                                                               725
<210> 10
   <211> 728
   <212> DNA
   <213> Streptococcus salivarius
<400> 10
   ttgtcatcaa ccatgtgtga agtttgatca tgtacatgac accaactgat acacggttat    60
   caaatggttc acctgtacgt ccatcgtaaa ggattgtctt agcatcacta tccatacctg   120
   cttcacgaac agtatcccag aggtcttctg agcttgcccc gtcaaagact ggtgttgcga   180
   tgtggatacc caagttacga gcagccatac caaggtgaag ttccataacc tgaccgatgt   240
   tcatacgtga tggcacccca agagggttca acatgatatc aactggtgta ccgtctggaa   300
   ggtaaggcat gtcttcaaca ggaacaatac gagaaacaac ccctttgtta ccgtgacgac   360
   cggccatctt atctccgacc ttaatcttac gtttttgagc gatgtaaaca cgaacaagca 420
   tgttaacacc tgattgcaat tcatcaccgt ttgcacgtgt gaagatttta acatcacgaa 480
   cgacaccatc accaccgtga ggtacacgga gtgaggtatc acgtacttca cgagatttat 540
   caccaaagat agcatggaga agacgttctt cagcagaaag gtctttttca cccttaggtg 600
   ttaccttacc aacaagaatg tcaccttctt taacctcagc accgatacgg ataataccca 660
   tttcgtcaag gtctttgaga gcttcttcac caacgtttgg caattcacgt gtaatttctt 720
   caggtcca 728
<210> 11
   <211> 725
   <212> DNA
   <213> Streptococcus pyogenes
<400> 11
   tgtcatcaac catgtggtga agtttgatca tatacatgac accaacggat acacggttgt 60
   caaatggttc accggtgcga ccatcataaa ggaccgtctt agcatcgcta tccataccag 120
   cttcacgaac agtgtcccaa aggtcttctg atgaagcccc gtcaaagaca ggtgttgcaa 180
   tgtgaatacc aagattacga gcagccatac caaggtgaag ttccataacc tgaccaatat 240
   tcatccgtga tggcacccca agagggttca acatgatgtc aactggtgtt ccgtctggaa 300
   ggtatggcat gtcttcaact ggtacaatac gtgaaacgac acccttgttt ccgtgacgac 360
   cggccatttt atctccgacc ttgattttac gtttttgagc gatgtaaaca cgcacaagca 420
   tattaacacc tgattgcaat tcatcgccgt tagcgcgtgt aaagattttc acatcacgaa 480
   cgataccatc accaccgtga gggacacgaa gtgaggtatc acgcacttca cgcgatttat 540
   ccccaaagat ggcgtgaagt aaacgttctt cagcagaaag gtctttttca cctttaggtg 600
   tgactttacc tactaagatg tcgccttctt taacctcagc accgatacgg ataatgccca 660
   tttcgtcaag gtctttgagg gcttcttcac caacatttgg gatttccgag tgattcttca 720
   gggca 725
<210> 12
   <211> 724
   <212> DNA
   <213> Streptococcus pneumoniae
<400> 12
   caaccatgtg gtggagtttg atcatgtaca tgactccgac agaaaacacg gttatcaaac 60
   ggttcaccag tacgtccatc gtaaaggatc gttttggcat cgctatccat acctgcttct 120
   ttaacagttg accaaagatc ttcagaactt gctccatcaa agactggtgt cgcgatgtga 180
   ataccaagag tacgagctgc cataccaagg tgaagctcca taacctgacc gatattcata 240
   cgtgatggta ccccaagtgg gttcaacatg atgtcgactg gagttccgtc tggaaggtaa 300
   ggcatgtctt ctacaggaac gatacgagag acaacccctt tgtttccgtg acgtccggcc 360
   attttatctc cgaccttaat cttacgtttt tgagcgatgt aaacacgaac caacatgtta 420
   acacctgatt gcaactcatc tccatttaca cgtgtaaaga tcttaacatc acgaacgaca 480
   ccatcggcac cgtgtggtac acgaagagaa gtatcacgca cttcacgaga cttgtctcca 540
   aagatagcgt gcaagagacg ttcttcagct gaaagatctt tctcaccctt aggtgttact 600
   ttacctacaa gaatatcacc ttctttaacc tcagcaccaa tacggataat cccatttcgt 660
   caaggtcttt gagggcatct tcaccaacgt tttggaattt cgcgagtgat ttcttcaggt 720
   ccaa 724
<210> 13
   <211> 694
   <212> DNA
   <213> Streptococcus oralis
<400> 13
   actcgtgaaa ttccaaacgt tggtgaagat gcccttaaag accttgacga aatgggtatt 60
   atccgtattg gtgctgaggt taaagaagga gatatccttg taggtaaagt cacacctaag 120
   ggtgaaaaag acctttctgc tgaagaacgt ctcttgcacg ctatcttcgg agacaagtct 180
   cgtgaagtgc gtgatacttc tcttcgagta cctcacggtg ccgatggtgt cgttcgtgat 240
   gttaagatct ttacacgtgc aaatggtgat gagttgcaat ctggtgtgaa tatgctggtt 300
   cgtgtctaca tcgctcaaaa acgtaagatc aagtcggaga taagatggcc ggacgtcacg 360
   gaaacaaagg ggttgtctct cgtatcgttc ctgtagaaga catgccttac cttccagatg 420
   gaactccagt cgatatcatg ttgaacccac ttggggtgcc atcacgtatg aatatcggtc 480
   aggttatgga actccacctt ggtatggcag cccgtactct tggtatccac atcgcaacac 540
   cagtctttga cggagcaagt tcggaagacc tttgggacac tgttaaagaa gcaggtatgg 600
   atagcgatgc caaaacaatc ctttacgatg gacgtacagg tgagccgttt gacaaccgtg 660
   tatcagttgg tgtcatgtac atgatcaaac tcca 694
<210> 14
   <211> 728
   <212> DNA
   <213> Streptococcus mutans
<400> 14
   tgtcatcaac catgtggtga agtttaatca tatacataac accaactgat acacgattat 60
   caaacggctc ccctgtgcga ccatcataaa gaacagtttt agcatcagaa tccataccgg 120
   cttcttttac tgtttcccaa agatcatcag aagttgctcc gtcaaagaca ggcgttgcaa 180
   tatgaatgcc caaattacga gcagccatac caagatggag ttccataact tgcccaatgt 240
   tcatccgtga tggcacccca agtggattaa gcatgatatc aacaggtgtt ccatctggaa 300
   gatatggcat atcttccact ggtacaatac gggaaacgac acccttgtta ccatgacgtc 360
   cggccatctt atctccgacc ttgattttac gtttttgagc gatataaaca cgaaccagca 420
   tgttaacacc tgattgaagt tcatctccat tagcacgtgt aaagattttc acatcacaaa 480
   caacaccgtc gccaccatga ggtacacgaa gagaagtatc acgaacttca cgtgatttgt 540
   caccaaaaat ggcatgcaag aggcgttctt ctgcagaaag atctttttct cctttaggag 600
   tcactttacc aactagaata tcaccttctt taacctcagc accaatgcga ataattccca 660
   tttcatcaag gtctttcagg gcatcttcac caacatttgg gatttcacgc gtaatttctt 720
   caggtcca 728
<210> 15
   <211> 730
   <212> DNA
   <213> Streptococcus mitis
<400> 15
   tgtcatcaac catgtggtgg agtttgatca tgtaacatga ctccgacaga aaacacggtt     60
   atcaaatggt tcacctgtac gtccatcgta aaggattgtt ttggcatcgc tatccatacc    120
   agcttcttta acagttgacc aaagatcttc agaacttgct ccgtcaaaga ctggtgttgc    180
   gatgtgaata ccaagagtac gagctgccat cccaaggtgg agttccataa cctgaccgat    240
   attcatacgt gatggcaccc caagtgggtt caacatgata tcgactggag ttccatctgg    300
   aaggtaaggc atatcttcta caggaacgat acgagagaca acccctttat ttccgtgacg     360
   tccggccatc ttatctccga ccttgatctt acgtttttga gcgatgtaga cgcgaaccag    420
   catgttgaca cctgattgca attcatctcc atttgcacgt gtaaagatct taacatcacg    480
   aaccacacca tcagctccgt gtggtacacg aagagaagtg tcacgtactt cacgagattt    540
   atctccgaag atagcgtgca agagccgttc ttcagctgaa aggtctttct cacccttagg    600
   tgttacttta cctacaagga tatccccttc tttaacctca gcaccgatac ggataatacc    660
   catttcgtca agatctttaa gggcatcttc cccaacgttt gggatttcac gagtaatttc    720
   ttcaggtcca                                                           730
<210> 16
   <211> 697
   <212> DNA
   <213> Streptococcus equinus
<400> 16
   cactcgcgaa attccaaacg ttggtgaaga agctcttaaa gaccttgacg aaatgggtat 60
   tatccgtatc ggtgctgaag ttaaagaagg tgacatcctt gtaggtaaag taacacctaa 120
   aggtgaaaaa gacctttctg ctgaagagcg ccttcttcac gcaatcttcg gtgataaatc 180
   acgtgaagtt cgtgatacat cacttcgtgt accacacggt ggagatggtg tcgttcgtga 240
   cgttaaaatc tttacacgtg caaacggtga tgaattacaa tcaggtgtta acatgctcgt 300
   tcgtgtttat atcgcacaaa aacgtaaaat caaagtcgga gataaaatgg ccggtcgtca 360
   cggtaacaaa ggggttgttt ctcgtgttgt tccagttgaa gacatgcctt atcttccaga 420
   cggaactcca gtcgatatca tgttgaaccc acttggggtg ccatctcgta tgaacatcgg 480
   acaagttatg gagcttcacc ttggtatggc tgctcgtaac cttggtattc acattgcaac 540
   accagtcttt gatggggcaa cttctgaaga cctttgggat acagttaacg aagctggtat 600
   ggctagcgac gctaagacag ttctttacga tggacgtact ggtgaaccat ttgataaccg 660
   tgtgtcagtt ggtgtcatgt acatgattaa acttcac 697
<210> 17
   <211> 731
   <212> DNA
   <213> Streptococcus constellatus
<400> 17
   agttgtcatc aaccatgtgt gcaatttaat catatacatg acaccgacag atacacggtt 60
   gtcaaacggc tcgcccgtac gaccatcata aagaatcgtc ttggcatcgc tatccatgcc 120
   tgcttcacga acagtatccc aaaggtcatc tgagcttgct ccgtcaaata ctggcgttgc 180
   tatgtggata ccaaggttgc gagcagccat accaaggtga agctccataa cctgtccgat 240
   attcatacgt gatggcaccc caagtgggtt caacatgatg tctactggtg ttccgtctgg 300
   aagataaggc atatcctcaa ctggaacgat acgggaaaca acccctttat ttccgtggcg 360
   tccggccatc ttatccccaa cgcggatctt tcgtttttga gcaatgtaaa cacgcaccaa 420
   catgttgaca ccagattgca attcatcacc gttcgcacga gtaaagattt tcacatcacg 480
   gacaacccca gcaccaccat gtggtacacg aagagatgtg tcacgtactt cacgagattt 540
   atcaccgaaa attgcatgaa gcaggcgttc ttcagcggat aagtcttttt cacctttcgg 600
   cgttacttta ccgacaagaa tgtcgccctc tttcacctca gcaccaatgc ggataattcc 660
   catttcgtca aggtctctta gcgcatcttc cccaacgttt ggaatttcgc gcgtaatttc 720
   ttcaggtcca a 731
<210> 18
   <211> 697
   <212> DNA
   <213> Streptococcus anginosus
<400> 18
   cacgcgcgaa attccaaacg tcggtgaaga tgctttgaga gaccttgacg aaacgggaat 60
   tatccgcatt ggtgctgagg taaaagaagg cgacattctt gtcggtaaag taacaccgaa 120
   aggtgaaaaa gacttatctg ctgaagaacg cctgcttcat gcaattttcg gtgataaatc 180
   tcgtgaagta cgtgatactt cccttcgtgt accacatggt ggtgcagggg ttgtccgtga 240
   tgtgaaaatc tttactcgtg cgaacggtga tgaattgcaa tctggtgtca acatgttggt 300
   acgtgtttac atcgctcaaa aacggaaaat ccgtgttggg gataagatgg ctggacgtca 360
   cggaaacaaa ggggttgttt cccgcattgt tccagttgag gatatgccgt atcttccaga 420
   tggaacacca gttgatatta tgttgaaccc acttggggtg ccatctcgta tgaatattgg 480
   tcaagttatg gagcttcacc tcggtatggc tgctcgcaac cttggcattc acattgcaac 540
   accagtattt gacggggcta gctcagatga tctttgggaa accgttcgtg aagctggcat 600
   ggatagcgat gctaagacaa tcctttatga tggccgtact ggtgagccat ttgataatcg 660
   tgtatccgtt ggtgtcatgt acatgatcaa actccac 697
<210> 19
   <211> 728
   <212> DNA
   <213> Streptococcus dysgalactiae
<400> 19
   tgtcatcaac catgtggtgg agtttaatca tgtacatgac accaacggat acacggttgt 60
   caaatggttc gccagtacgt ccatcataaa ggaccgtctt agcatcgcta tccataccag 120
   cttcacgaac agtgtcccaa aggtcttctg atgaagcccc gtcaaagaca ggtgttgcaa 180
   tgtgaatacc aagattacga gcagccatac caaggtgaag ttccataacc tgaccaatgt 240
   tcatccgtga tggcacccca agagggttca acatgatgtc aactggtgtt ccatctggaa 300
   ggtatggcat gtcttcaact ggtacaatac gtgaaacgac acccttgttt ccgtgacgac 360
   cagccatttt atctccgact ttgatcttac gtttttgagc aatgtaaaca cgcacaagca 420
   tattaacacc tgattgcaat tcatcgccgt tagcgcgtgt aaagattttc acatcacgaa 480
   cgataccatc accaccgtga ggtacacgaa gggacgtatc acgaacttca cgtgatttat 540
   ctccaaagat ggcatgcaag agacgctctt cagcagaaag gtctttttca cctttaggtg 600
   tgactttacc tactaagatg tcgccttctt taacctcagc accgatacgg ataattccca 660
   tttcgtcaag gtctttgagc gcttcttcac caacgtttgg aatttcgcgg gtgatttctt 720
   caggtcaa 728
<210> 20
   <211> 728
   <212> DNA
   <213> Streptococcus bovis
<400> 20
   tgtcatcaac catgtggtga agtttgatca tgtacatgat accaacagag acacgattat      60
   caaatggttc acctgtacga ccgtcataaa gaactgtctt agcgtcgcta tccataccag     120
   cttcacgaac agtatcccaa aggtcttctg aagttgcccc gtcaaagact ggagttgcaa     180
   tgtgaatacc gaggttacga gctgccatac caaggtgaag ttccataact tgtccgatat     240
   tcatacgaga tggcacccca agagggttca acatgatatc aactggagtt ccgtctggaa     300
   gatatggcat gtcttcaaca ggaacgatac gagaaacaac ccctttgttt ccgtgacgac     360
   cggccatttt atctccgact ttgattttac gtttttgtgc aatgtaaaca cgaacgagca     420
   tgttgacacc tgattgcaat tcatcaccgt tagcacgtgt gaagatttta acatcacgaa     480
   caacaccgtc tccaccgtgt ggcacacgaa gtgatgtatc acgtacttca cgagatttat     540
   caccgaagat tgcgtgaaga aggcgttctt cagcagaaag gtctttttca cctttaggtg     600
   ttactttacc tacaaggata tcaccttctt taacttcagc accgatacgg ataataccca     660
   tttcgtcaag gtctttaaga gcttcttcac caacgtttgg aatttcgcga gtgatttctt     720
   caggtcaa                                                              728
<210> 21
   <211> 728
   <212> DNA
   <213> Streptococcus acidominimus
<400> 21
   ttgtcatcaa ccatgtggtg gagcttaatc atgtacatga caccaacaga cacacggtta      60
   tcaaatggtt caccagtacg accatcataa agaatcgttt tagcatcgct gtccattcct     120
   gcctctttaa cagttgacca gagatcctct gagctcgcac catcgaaaac cggtgttgcg 180
   atatggatac ccaagttacg agcagccata cccaagtgca gttccataac ctgaccaata 240
   ttcatacgag atggcacccc aagtgggttc aacatgatgt caactggtgt tccatctgga 300
   agatatggca tgtcttcaac tggtacaata cgagaaacga cacccttgtt accgtgacga 360
   ccggccatct tatctccgac cttaatcttg cgtttttgag cgatatacac acgtaccagc 420
   atattaacac cagactgtag ctcatcacca ttagcacgcg taaagatttt cacatcacga 480
   acaacaccat ctgcaccgtg tggcacacgt agagaggtat cacgtacttc acgtgatttg 540
   tcaccgaaga tagcatgcaa gagacgctcc tcagcagaaa gatctttttc accttttggt 600
   gtcaccttac caacaagaat atcgccttct ttaacttctg caccgatacg gataataccc 660
   atttcgtcaa ggtctttgag ggcttcttca ccaacgtttg gaatttcacg agtaatttct 720
   tcaggtca 728
<210> 22
   <211> 733
   <212> DNA
   <213> Streptococcus agalactiae
<400> 22
   tgagttgtca tcaaccatgt ggtgaagttt gatcatgtac atgacaccaa ctgacacacg 60
   gttatcgaat ggttcaccag tacgaccatc ataaagaaca gtcttagcat ctgaatccat 120
   acctgcttct tgaacagttt cccaaaggtc ttctgaagaa gccccatcaa agactggcgt 180
   tgcaatatga atacctaaat tacgagcagc catacctaaa tgaagctcca taacttgtcc 240
   gatattcata cgtgatggca ccccaagtgg gttcaacatg atatcaactg gcgttccatc 300
   tggtaagtaa ggcatatctt caacaggaac aatacgtgag acgacacctt tgtttccgtg 360
   acgaccggcc atcttatcac cgactttgat tttacgtttt tgagcgatat aaacgcggac 420
   aagcatatta acacctgatt gcaattcatc accatttgca cgagtaaaga ttttaacgtc 480
   acgaactact ccatcgccac cgtgaggtac acgtagtgaa gtatcacgaa cttcacgtga 540
   tttatcacca aaaatggcat gcaagagacg ttcttcagca gataagtcct tttcaccctt 600
   aggtgttacc ttaccaacaa gaatgtcacc ttcttttacc tcagcaccaa tgcggataat 660
   tcccatttca tcgagatcac gtagtgaatc ttcaccaaca ttttggattt cacgagtaat 720
   ttcttcaggt cca 733
<210> 23
   <211> 714
   <212> DNA
   <213> Streptococcus difficilis
<400> 23
   ttgtcatcaa ccatgtggtg aagtttgatc atgtacatga caccaactga cacacggtta      60
   tcgaatggtt caccagtatg accatcataa agaacagtct tagcatctga atccatacct     120
   gcttcttgaa cagtttccca aaggtcttct gaagaagccc catcaaagac tggcgttgca     180
   atatgaatac ctaaattacg agcagccata cctaaatgaa gctccataac ttgtccgata     240
   ttcatacgtg atggcacccc aagtgggttc aacatgatat caactggcgt tccatctggt     300
   aaataaggca tatcttcaac aggaacaata cgtgagacga cacctttgtt tccgtgacga     360
   ccggccatct tatcaccgac tttgatttta cgtttttgag cgatataaac gcggacaagc     420
   atattaacac ctgattgcaa ttcatcacca tttgcacgag taaagatttt aacgtcacga     480
   actactccat cgccaccgtg aggtacacgt agtgaagtat cacgaacttc acgtgattta     540
   tcaccaaaaa tggcatgcaa gagacgttct tcagcagata agtccttttc acccttaggc     600
   gttaccttac caacaagaat gtcaccttct tttacctcag caccaatgcg gataattccc     660
   atttcatcga gatcacgtag tgaatcttca ccaacatttg gaatttcacg agta           714
<210> 24
   <211> 728
   <212> DNA
   <213> Streptococcus intermedius
<400> 24
   tgtcatcaac catgtggtga agcttaatca tgtacatgac accaacggac acacggttat    60
   caaacggttc gccagtacgt ccatcataaa ggattgtctt agcatcgcta tccatacctg   120
   cttcacgaac ggtttcccaa agatcatctg agctagctcc gtcaaagact ggcgttgcaa   180
   tgtggatacc aaggttgcga gcagccatac cgaggtgcaa ttccataact tgtccgatat   240
   tcatacgtga cggcacccca agaggattca acatgatatc aactggtgtc ccgtctggaa   300
   gatacggcat atcctcaact ggaacaatgc gggaaacaac ccctttgttt ccgtggcgtc   360
   cggccatctt atctccaacg cggattttcc gtttttgagc gatataaaca cgtaccaaca   420
   tgttgacacc ggattgcaat tcatcaccgt tcgcacgagt aaagattttt acatcacgga   480
   caacacctgc accaccgtgt ggtacacgaa gggaggtatc acgcacttca cgagacttat   540
   caccaaaaat tgcatgaagc aggcgttctt cagcggataa atctttttca cctttcggcg   600
   ttactttacc gacaagaatg tcgccttctt ttacctcagc accaatgcgg ataattccca   660
   tctcgtcaag gtctctcaaa gcatcttccc cgacgtttgg aatttcgcgc gtgatttctt   720
   caggtcca                                                            728
<210> 25
   <211> 728
   <212> DNA
   <213> Streptotoccus equi
<400> 25
   tgtcatcaac catgtggtga agcttaatca tatacatgac accaactgac acacgattat    60
   caaacggctc accagtacgg ccatcataaa gaacagtctt agcatcgcta tccatacctg   120
   cttcacgaac agtttcccaa aggtcctcag acgtagctcc gtcaaagacc ggtgttgcga   180
   tatggatacc caaattacga gcagccatac ctaggtgaag ctccataacc tgtccaatgt   240
   tcatacgaga cggcacccca agagggttca gcatgatgtc aacaggggtt ccgtctggca   300
   gatatggcat atcctcaacc ggtacaatac gtgagacgac acccttgtta ccatgacgcc   360
   cggccatttt atctccgacc ttgattttac gcttttgagc aatgtaaaca cgcaccagca   420
   tattaacacc tgattgaagc tcatcaccat ttgcgcgtgt aaagatcttc acatcacgta   480
   caatcccgtc accaccatga ggaacacgta acgaggtatc acgaacctca cgtgatttat   540
   caccaaagat agcatgcagg agacgttctt cagcagaaag gtctttttca cccttaggag   600
   ttaccttacc aacaagaata tcgccttcct tgacctctgc accgatacgg ataataccca   660
   tttcatcaag gtccttgagg gcttcttcac caacgtttgg cacttcacgt gtgatttctt   720
   caggtcca                                                            728
<210> 26
   <211> 697
   <212> DNA
   <213> Enterococcus gallinarum
<400> 26
   cactcgtgaa atcccgaatg tcggggaaga cgcattgaaa gatctagacg aaatgggtat    60
   catccgcatt ggtgcggaag tcaaagatgg cgatctgttg gttggtaaag taacgcctaa   120
   aggggtaacg gaactatctg cagaagaacg cttgcttcat gcaatctttg gtgaaaaagc   180
   ccgcgaagtc cgcgatactt ctctgcgcgt acctcacggt ggtggcggaa tcgtccatga   240
   tgtgaaaatc tttacccgcg aagctggcga tgaattgtca ccaggtgtca atatgctcgt   300
   tcgcgtgtat atcgttcaaa aacggaaaat ccatgaaggg gataaaatgg ccggccgtca   360
   cggaaataaa ggggtcgttt ctcgcattat gccagaagaa gacatgcctt tcttaccaga   420
   cggtacacca gttgatatca tgttgaaccc attaggggtg ccttcacgga tgaacattgg   480
   acaagtattg gaattacact taggaatggc tgcccgccaa ttaggaatcc acgtggctac   540
   accagtcttt gatggtgcca gcgatgaaga tgtctgggca acagttgcag aagccggcat   600
   ggctagcgac gccaaaaccg ttttgtatga tggccgtact ggagaaccat ttgatggtcg   660
   aatctccgta ggtgtcatgt atatgatcaa attggcc                            697
<210> 27
   <211> 727
   <212> DNA
   <213> Enterococcus casseliflavus
<400> 27
   tgtcatcaac catgtgggcc aatttgatca tgtacatgac accaacggag atgcggccat    60
   caaatggttc gccggtacgt ccgtcgtaaa gcactgtttt ggcatcgctg gccattcctg   120
   cttcagcaac cgttgcccaa acatcttcat cgctggctcc atcaaagact ggtgttgcca   180
   cgtgaatgcc taattgacgc gcagccattc ctaagtgtaa ctctaatact tgtccaatgt   240
   tcatccgaga aggtacccct aatgggttca gcatgatatc gactggtgtg ccatctggta   300
   agaaaggcat gtcttcttct ggcataatgc gagaaacgac ccctttgttt ccgtgacgtc   360
   cggccatttt atccccttca tggattttcc gtttttgaac gatataaacg cgaaccagca   420
   tgttcacacc tggtgacaat tcatcgccag cttcgcgggt aaagattttg acatcgtgga   480
   cgattccgcc gccgccgtga ggcacgcgta gagaagtgtc acgcacttcg cgggcttttt   540
   caccaaagat tgcgtgcaac aaacgctctt ctgctgaaag ttccgttacc ccttttggcg   600
   tgactttccc aacaagcaga tcgccatctt tgacttccgc accaatgcgg ataatgccca   660
   tttcgtctag gtctttcaac gcgtcttccc aacgttcggg atttcgcgag tgatttcttc   720
   aggtcca                                                             727
<210> 28
   <211> 721
   <212> DNA
   <213> Enterococcus saccharolyticus
<400> 28
   tgtcatcaac catgtgggca agtttaatca tgtacattac cccaacagag atacgaccat    60
   cgaatggttc acccgtacgt ccgtcataaa gaacagtttt cgcatcgcgc gccatgcccg   120
   cttcgcgaac tgtttcccat acgtcatcat ctgatgcacc atcaaatact ggtgtagcta   180
   catggatgcc taactgacgt gcagccatcc ctaagtgtaa ttccaatact tgtccgatgt   240
   tcatacgaga tggtactcct agtgggttca acatgatatc aactggtgtg ccgtctggta 300
   agaatggcat gtcttcttct ggcataatgc gagagacaac ccctttgtta ccatgacgtc 360
   ccgccatttt atctccttcg tgaatcttac gtttttgcac gatataaaca cgaactaaca 420
   tgttcacacc tggagataat tcgtcgcctg cttcacgggt aaagatttta acatcgtgaa 480
   cgataccgcc accgccgtga ggaacacgta atgatgtatc acgtacttca cgtgcttttt 540
   caccgaagat tgcgtgcaat agacgttctt ctgcagataa ttcggttacc cctttaggag 600
   tgactttacc tactaataag tcgccatctt gtacttcggc accgatacgg ataataccca 660
   tttcgtctaa gtcttttaat gcgtcttccc caacgttagg aatttcgcgt gtattcttca 720
   g 721
<210> 29
   <211> 727
   <212> DNA
   <213> Enterococcus faecium
<400> 29
   tgtcatcaac catgtgagca agtttgatca tgtacatcac accgacagac acacgtccat 60
   caaatggttc acctgtacgt ccgtcgtaca gaacagtttt cgcatcgctg gccataccgg 120
   cttcacgaac tgtttcccat acgtcttcat cacttgcacc atcaaatact ggcgttgcta 180
   cgtggatacc taactgacgt gcagccatac ccaagtgtaa ttccaatact tgcccgatgt 240
   tcatacgtga aggcacccct aaaggattca gcatgatatc gattggtgtt ccatcaggta 300
   ggaatggcat atcttcttcc ggcataatac gggatacaac ccctttattt ccgtgacgac 360
   cggccatttt atccccttca tggattttac gtttttgaac gatataaaca cgaactaaca 420
   tgtttacgcc tggtgacaat tcatctccag cttcacgagt aaagattttc acatcgtgaa 480
   cgataccgcc gccgccatgt ggtacacgta atgatgtatc gcggacttca cgagcttttt 540
   cgccaaagat cgcatgcaat agacgttctt ctgcagataa ttctgttacc ccttttggcg 600
   tgactttccc tacaagcaaa tcgccatctt ggacttctgc accaatacgg atgataccca 660
   tttcgtctaa atcttttaat gcgtcttccc gacattaggg atttcgcgtg tgatttcttc 720
   aggtcca 727
<210> 30
   <211> 725
   <212> DNA
   <213> Enterococcus faecalis
<400> 30
   tgtcatcaac catgtgggct aatttaatca tatacatgac accaacggaa atacggttat 60
   caaatggttc acctgtacgt ccatcgtaaa gaactgtttt agcatcgcta gccataccag 120
   cttcacgaac agtttcccaa acgtcttcat cggttgcccc atcgaaaaca ggtgttgcga 180
   cgtgaatacc taattggcga gcagccatac ctaagtgtaa ttcaagtact tgtccgatat 240
   tcatacgaga aggtacccct aatgggttca acatgatatc aacaggtgtt ccgtcaggta 300
   agaatggcat atcttcttcc ggcataatac gggaaacaac ccctttattt ccgtgacgtc 360
   ccgccatttt atctccttcg tgaattttac gtttttgaac gatatagaca cgaactaaca 420
   tgttgacacc tggtgataat tcatcgccag cttcacgagt aaagattttc acatcatgaa 480
   cgataccgcc gccaccgtga ggtacacgga gagacgtatc acgaacttcg cgggcttttt 540
   ccccgaagat tgcgtgtaat aaacgttctt ctgcagataa ttctgtgacc cctttaggtg 600
   tgactttccc aactagtaag tcgccatctt gaacttcagc accaatgcgg ataatcccca 660
   tttcgtctaa gtctttcaac gcgtcttccc aacgtttgga atttcacggg tatttcttca 720
   ggtca 725
<210> 31
   <211> 570
   <212> DNA
   <213> Enterococcus avium
<400> 31
   gtccatcata aagaacggtc ttagcatctg ctgccatacg agcttcacga actgtttccc 60
   aaacatcgct atcttgcgca ccatcgaaga ctggtgtcgc aacatggata cctagttggc 120
   gagccgccat tcccaagtgt aattccaaca cttgtccgat gttcatccga gatggcacac 180
   ctaatgggtt caacatgata tcaactggcg taccgtctgg taagaaaggc atgtcttctt 240
   ctggcataat gcgagaaacg acccctttat ttccgtgacg gccggccatt ttatcccctt 300
   catgaatctt acgtttttgc acgatgtaca cgcgcactaa catatttaca cctggagata 360
   attcatcgcc tgcttcacga gtaaagatct tcacatcgtg aacgatcccg ccgccaccat 420
   gcggtacacg aagagatgta tcacgaactt cacgagcctt ttcaccaaag atcgcatgca 480
   acaaacgttc ttcagctgat aattctgtta cccctttagg agtgacttta ccaactaata 540
   aatcaccatc atgaacttca gcaccaatac 570
<210> 32
   <211> 732
   <212> DNA
   <213> Abiotrophia defectiva
<400> 32
   gaagttgtca tcaaccatgt gggccaactt aatcatgtac ataaccccaa cagagacttt    60
   acggtcaaat ggttcaccgg ttcgaccatc atataagata gtcttagcgt cagcttctaa   120
   gccggcttcc ttaactgttt cccagacatc ttcttcacta gcaccgtcaa agacaggtgt   180
   tgcaatcttg atgcccattt cgcgagcagc catccccaag tgtaactcta ggacttgccc   240
   gatgttcata cgggatggaa cccctaatgg gttcaacatg atatcaactg gggtaccatc   300
   tggtaagaat ggcatatctt cttccggcat gataagggag acaacccctt tgttaccgtg   360
   acgaccggcc atcttatccc cttcattgat tttacgtttt tgtacgatgt agacgcggac   420
   tagcttgttg acacctggtg ccaattcgtc gccagcttcg cgggtaaaga ttttaacgtc   480
   gtggacaatc ccgcccccgc cgtgtggcac acgcaaggaa gtatcacgta cttcacgcgc   540
   cttctcaccg aagatagcat ggagcaagcg ttcttccgca gacaactcgg tcacaccttt   600
   tggtgttacc ttaccaacta agatatcgcc gtcttttact tccgccccga tacagataat   660
   cccgtcttgg tctaagtact tgagggcatc ttcggacacg tttggaattt cgcgtgtaat   720
   ttcttcaggt ca                                                        732
<210> 33
   <211> 727
   <212> DNA
   <213> Gemella morbilorum
<400> 33
   tgtcatcaac catgtgtgca agtttatcat gtacattacc cctacagata cacggctatc    60
   aaatggctca cctgtacgtc cgtcataaag aactgtctta gcatctttag ccattccagc   120
   ttccgcaact gtagaccaaa catcttcatc agtagcacca tcgaatactg gtgtagctac   180
   gtggattcca agttgtttag cagccatacc taagtgtagc tctaatactt gtccaatgtt   240
   catacgagat ggaaccccaa gtgggtttaa cattacgtca actggtgtac catctggtag   300
   gtaaggcata tcttcttctg gtaagatatt tgagataacc cctttgttac cgtgacgacc   360
   ggccatttta tctcctacac gaattttacg tttttggacg ataaatacac gaacaagttc   420
   atttacaccg ttaggtaatt cagcaccatc ttcacgttta aagattttaa catcagcaac   480
   tactccatca gcaccgtgag gtacacgtaa tgaagtatca cgtacttctt tagatttagc   540
   tccaaagata gcatataata atttttcttc tggagtttgt tcagttaatc ctttcggtgt   600
   aactttacct actaaaatat ctccatcttt aacttcagcc ccaatacgaa tgattcctcg   660
   tgcatctaag tttctaagtg cattttcacc ctacgtttgg aatctcacga gtaatttctt   720
   caggtca                                                             727
<210> 34
   <211> 726
   <212> DNA
   <213> Gemella haemolysans
<400> 34
   tgtcatcaac catgtgtgca agtttaatca tgtacattac ccctacagat acacggctat     60
   caaatggctc acctgtacgt ccgtcataaa gaactgtctt agcatcttta gccattccag    120
   cttccgcaac tgtagaccaa acatcttcat cagtagcacc atcgaatact ggtgtagcta    180
   cgtggattcc aagttgttta gcagccatac ctaagtgtag ctctaatact tgtccaatgt    240
   tcatacgaga tggaacccca agtgggttta acattacgtc aactggtgta ccatctggta    300
   ggtaaggcat atcttcttct ggtaagatat ttgagataac ccctttgtta ccgtgacgac    360
   cggccatttt atctcctaca cgaattttac gtttttggac gataaataca cgaacaagtt    420
   catttacacc gttaggtaat tcagcaccat cttcacgttt aaagatttta acatcagcaa    480
   ctactccatc agcaccgtga ggtacacgta atgaagtatc acgtacttct ttagatttag    540
   ctccaaagat agcatataat aatttttctt ctggagtttg ttcagttaat cctttcggtg    600
   taactttacc tactaaaata tctccatctt taacttcagc cccaatacga atgattcctc    660
   gtgcatctaa gtttctaagt gcattttcac ctacgtttgg aatctcacga gtattcttca    720
   ggtcca                                                               726
<210> 35
   <211> 719
   <212> DNA
   <213> Granulicatella adjacens
<400> 35
<210> 36
   <211> 21
   <212> DNA
   <213> amorce
<400> 36
   agacggacct tctatggaaa a 21
<210> 37
   <211> 20
   <212> DNA
   <213> amorce
<400> 37
   ggacacatac gaccatagtg 20
<210> 38
   <211> 21
   <212> DNA
   <213> amorce
<400> 38
   gttgtaacct tcccawgtca t 21
<210> 39
   <211> 20
   <212> DNA
   <213> amorce
<400> 39
   gtcttcwtgg gygatttccc 20
<210> 40
   <211> 21
   <212> DNA
   <213> amorce
<220>
   <221> misc_feature
   <222> (8)..(8)
   <223> n représente i
<400> 40
   accgtggngc wtggttrgaa t 21
<210> 41
   <211> 20
   <212> DNA
   <213> amorce
<400> 41
   aaccaattcc gyatyggtyt 20
<210> 42
   <211> 20
   <212> DNA
   <213> amorce
<220>
   <221> misc_feature
   <222> (3)..(3)
   <223> n représente i
<400> 42
   agngggttta acatgatgtc 20
<210> 43
   <211> 20
   <212> DNA
   <213> amorce
<220>
   <221> misc_feature
   <222> (3)..(3)
   <223> n représente i
<400> 43
   agngcccaaa cctccatctc 20
<210> 44
   <211> 21
   <212> DNA
   <213> amorce
<400> 44
   ctccaagtga acagatgtgt a 21
<210> 45
   <211> 26
   <212> DNA
   <213> amorce
<400> 45
   ttaccaaact taattgagat tcaaac 26
<210> 46
   <211> 22
   <212> DNA
   <213> amorce
<400> 46
   agtatttatg ggtgatttcc ca 22
<210> 47
   <211> 26
   <212> DNA
   <213> amorce
<400> 47
   ggacgttata aaatcaacaa aaaatt 26
<210> 48
   <211> 23
   <212> DNA
   <213> amorce
<400> 48
   agttataacc atcccaagtc atg 23
<210> 49
   <211> 23
   <212> DNA
   <213> amorce
<400> 49
   tgaagtttat catcaaccat gtg 23
<210> 50
   <211> 18
   <212> DNA
   <213> amorce
<400> 50
   cccaaaacgt tgtccacc 18
<210> 51
   <211> 20
   <212> DNA
   <213> amorce
<400> 51
   aaccaagcyc ggttaggrat 20
<210> 52
   <211> 25
   <212> DNA
   <213> amorce
<220>
   <221> misc_feature
   <222> (15)..(15)
   <223> n représente i
<400> 52
   atgttgaacc cactnggggt gccat 25